(19) **Europäisches Patentamt / European Patent Office / Office européen des brevets**

(11) **EP 1 453 562 B1**

(12) # EUROPEAN PATENT SPECIFICATION

(45) Date of publication and mention
of the grant of the patent:
**15.02.2012 Bulletin 2012/07**

(21) Application number: **02799203.1**

(22) Date of filing: **04.12.2002**

(51) Int Cl.:
**A61M 11/06** (2006.01)

(86) International application number:
**PCT/US2002/038492**

(87) International publication number:
**WO 2003/049791 (19.06.2003 Gazette 2003/25)**

(54) **MEDICAL DEVICE FOR INHALATION OF AEROSOLIZED DRUG WITH HELIOX**

MEDIZINISCHE VORRICHTUNG ZUR INHALATION VON AEROSOLISIERTEM ARZNEIMITTEL MIT HELIOX

DISPOSITIF MEDICAL PERMETTANT L'INHALATION D'UN MEDICAMENT EN AEROSOL AVEC DE L'HELIOX

(84) Designated Contracting States:
**AT BE BG CH CY CZ DE DK EE ES FI FR GB GR IE IT LI LU MC NL PT SE SI SK TR**

(30) Priority: **05.12.2001 US 337609 P**

(43) Date of publication of application:
**08.09.2004 Bulletin 2004/37**

(73) Proprietor: **NuPharmx, LLC**
**Cumming, GA 30040 (US)**

(72) Inventors:
• **HALL, Jesse, B.**
**Chicago, IL 60615 (US)**
• **KRESS, John, P.**
**Hinsdale, IL 60521 (US)**
• **MORGAN, Sherwin, E.**
**Park Forest, IL 60466-4108 (US)**
• **PING, Jeffrey, H.**
**Cumming, GA 30040 (US)**
• **WARNER, W., Randolph**
**Punta Gorda, FL 33950 (US)**

(74) Representative: **Weiss, Wolfgang et al**
**Weickmann & Weickmann**
**Patentanwälte**
**Postfach 86 08 20**
**81635 München (DE)**

(56) References cited:
**WO-A-98/16271          US-A- 5 586 551**
**US-A1- 2001 035 181**

• **FW MOLER, CE JOHNSON, C VAN LAANEN, JM PALMISANO ...: "CONTINUOUS VERSUS INTERMITTENT NEBULIZED TERBUTALINE: PLASMA LEVELS AND EFFECTS" AM J. RESPIR. CRIT. CARE MED, vol. 151, no. 3, March 1995 (1995-03), pages 602-606, XP009007322**
• **DATABASE MEDLINE [Online] US NATIONAL LIBRARY OF MEDICINE (NLM), BETHESDA, MD, US; January 1999 (1999-01) HESS D R ET AL: "The effect of heliox on nebulizer function using a beta-agonist bronchodilator." Database accession no. NLM9925082 XP002234208 & CHEST. UNITED STATES JAN 1999, vol. 115, no. 1, January 1999 (1999-01), pages 184-189, ISSN: 0012-3692**

EP 1 453 562 B1

## Description

## Background of the Invention

This invention is generally in the field of devices for improving the delivery of drugs to the lungs of a patient in need thereof. More specifically, the invention relates to a device that provides an aerosolized drug for inhalation with heliox.

There is a large and growing need for the effective treatment of respiratory ailments. There is tremendous room for improvement over current therapies, which often deliver suboptimal doses and/or waste significant amounts of drug in the delivery process.

The use of heliox, a mixture of helium and oxygen, has long been considered for use in the treatment of respiratory ailments. For example, the use of helium in the treatment of asthma dates back to 1934 when Barach reported improvement in patients presenting with acute asthma exacerbations as well as upper airway obstructive lesions (Barach, Proc. Soc. Exp. Biol. Med 32:462-64 (1934)). Most studies of heliox use during acute asthma exacerbations have looked at helium gas itself as a means of decreasing airways resistance, improving flow and potentially averting respiratory failure or facilitating mechanical ventilatory support. These studies have measured changes in endpoints such as peak expiratory flow rate, pulsus paradoxus (Manthous, et al., "Heliox improves pulsus paradoxus and peak expiratory flow in nonintubated patients with severe asthma" Am. J. Respir. Crit. Care Med. 151:310-14 (1995)), peak airway pressure during mechanical ventilation (Kass & Castriotta, "Heliox therapy in acute severe asthma" Chest 107:757-60 (1995)) and arterial $PaCO_2$ and pH (Gluck, et al., "Helium-oxygen mixtures in intubated patients with status asthmaticus and respiratory acidosis" Chest 98:693-98 (1990)) during a time when a patient is actively breathing heliox.

Heliox does not possess bronchodilator or anti-inflammatory properties, but can reduce the work of breathing, due to heliox's lower density compared to air. The density of helium is one-seventh that of nitrogen, and this lower density beneficially reduces the pressure gradient associated with a given flow rate through turbulent airways (Ball, et al., Clin. Intensive Care, 12(3):105-13 (2001)). That is, the turbulent or transitional gas flow in portions of the upper airway can be made laminar with heliox where it would be turbulent with air. Consequently, the heliox can reduce airway resistance ($R_{aw}$) to 28-49% of that measured with air in normal subjects (Manthous, et al., Respiratory Care, 42(11): 1034-42 (1997)). It is theorized that the use of heliox also may advantageously increase the diffusional transport of oxygen in peripheral airways and alveoli. See, e.g., Nie, et al., Am. J. Physiol. Heart Circ. Physiol, 280:H1875-1881 (2001). These properties presumably decrease the work of breathing, providing an effective, albeit temporary benefit until more definitive pharmacological therapy has time to take effect.

Inhaled $\beta_2$ agonists such as albuterol are a mainstay of therapy for asthmatic patients suffering from acute exacerbations. Such medications are often delivered by jet nebulization using air or oxygen as the driving gas. The use of heliox as a driving gas for an aerosolized drug, such as albuterol, also has been contemplated. The theory behind replacing the oxygen with heliox is that heliox reduces turbulent flow in the upper airways, that turbulence is at least partially responsible for premature deposition of aerosol in medical device conduits or patients' upper airway, and that therefore drugs inhaled with heliox should be delivered to more distal airways with greater efficiency (Manthous, et al., Respiratory Care, 42(11):1034-42 (1997)).

Certain limited studies have been conducted on the use of heliox as a delivery gas. Habib et al., "Effect of helium-oxygen on delivery of albuterol in a pediatric volume cycled lung model" Pharmacotherapy 19:143-49 (1999) discloses that heliox 70:30 improved albuterol delivery compared to oxygen nebulizer treatments in a pediatric lung model. Goode et al., "Improvement in aerosol delivery with helium-oxygen mixtures during mechanical ventilation" Am J Respir Crit Care Med 163:109-14 (2001) discloses similar findings in a lung model of mechanical ventilation, whether albuterol was delivered by metered dose inhaler or nebulizer. Goode also discloses that the best delivery is achieved using 100% $O_2$ at a low flow rate to drive the nebulizer into a heliox (80:20) ventilation circuit. In addition, Svartengren et al., Experimental Lung Research, 15:575-85 (1989) discloses the delivery of 3.7 micron diameter (aerodynamic), radiolabeled TEFLON™ particles to the alveoli of human subjects with and without induced bronchoconstriction. The particles were inhaled in air or heliox (80:20) using flow rates of 0.5 and 1.2 L/s. Significantly greater deposition was reported with the heliox gas. Similar results were obtained in an identical study using stable asthmatic subjects. (Anderson, et al., Am. Rev. Respir. Dis. 147:524-28 (1993)).

Several clinical studies, however, have failed to show a statistically significant advantage to using heliox over air in the nebulization of aerosolized medications, particularly for acute severe asthma exacerbation or acute chronic obstructive pulmonary disease (COPD) exacerbation. For example, Henderson et al., Ann. Emerg. Med., 33:141-46 (1999) concludes that "despite its ability to decrease turbulent flow in airways and to reach distal pulmonary tissue, heliox had no clinically significant advantage over standard therapy in the treatment of mild to moderate asthma." As another example, Ball, et al., Clin. Intensive Care, 12(3):105-13 (2001) indicates that "overall, it appears that enhanced delivery of bronchodilators is unlikely to be the explanation for the positive findings in the asthma trials." As yet another example, deBoisblanc et al., Crit. Care Med. 28(9):3177-80 (2000) concludes that the "use of heliox as a driving gas for the updraft nebulization of bronchodilators during the first 2 hrs of treatment of an acute COPD exacerbation failed to

improve FEV$_1$ faster than the use of air. The faster improvement in FEF$_{25-75}$ during the first 2 hrs of treatment was small and of uncertain clinical significance." It is not clear from the literature why heliox failed in these studies to demonstrate enhanced drug delivery or enhanced treatment of acute asthmatic or COPD exacerbations, as would have been theoretically expected from gas flow physics.

It therefore would be desirable to develop improved and effective device and methods for delivering aerosolized medications by inhalation with heliox, to capitalize on the physical property benefits offered by heliox. It would also be desirable to develop improved methods for treating acute asthma, COPD, cystic fibrosis, and other respiratory obstructive diseases and disorders.

The publication by FW MOLER, CE JOHNSON, C VAN LAANEN, JM PALMISANO: "CONTINUOUS VERSUS INTERMITTENT NEBULIZED TERBUTALINE: PLASMA LEVELS AND EFFECTS", AM J. RESPIR. CRIT. CARE MED, vol. 151, no. 3, March 1995 (1995-03), pages 602-606 shows a medical device suitable for use with Heliox.

## Summary of the Invention

An improved medical device has been developed for the pulmonary administration of a drug to a patient. The device overcome, or at least partially alleviate, the problem of entrainment of air from the environment, which decreases the concentration of inspired helium, thereby increasing the density of the inhaled gas, which may limit the effectiveness of heliox. The present medical device therefore provides delivery of a high concentration of helium and aerosolized drug to a patient's lungs.

In one aspect, a medical device as defined in claim 1 is provided for using heliox gas to deliver a drug to the lungs of a patient in need thereof. The device comprises (a) an aerosolization subassembly which comprises: (i) a gas inlet for connection to a first heliox gas source, (ii) a drug reservoir for containing a drug to be administered, (iii) an atomization means for forming an aerosol of particles or droplets dispersed in a heliox driving gas received from the first heliox gas source, wherein the particles or droplets comprise the drug, and (iv) a discharge outlet for discharging the aerosol; (b) a gas mask which can be secured over a patient's mouth and nose, the gas mask comprising a source gas aperture; (c) a secondary gas inlet for connection to a second heliox gas source; and (d) a branched conduit means which comprises a first inlet port, a second inlet port, and an outlet port, wherein the first inlet port is in communication with the discharge outlet of the aerosolization subassembly, the second inlet port is in communication with the secondary gas inlet, and the outlet port is in communication with the source gas aperture of the gas mask; wherein the device is operable as a closed system to prevent dilution of the aerosol with ambient air before and during inhalation of the aerosol by the patient. The device includes a reservoir bag in fluid communication with the second inlet port of the branched conduit means.

The device further includes a source of compressed heliox gas connected to the gas inlet of the nebulizer, or to the secondary gas inlet, or both. In one embodiment, the source of compressed heliox gas comprises a tank coupled to a single regulator valve having two discharge outlets. In one embodiment, the single regulator valve can provide identical flow rates of heliox through each of the two discharge outlets.

In various embodiments, the aerosolization subassembly can comprise a jet nebulizer or pneumatic nebulizer, an ultrasonic nebulizer, or an electrostatic nebulizer. Alternatively, the aerosolization subassembly can be adapted for dry powder drug delivery. In a preferred embodiment, the device produces an aerosol wherein greater than 55 wt% of the drug is in the form of particles having a diameter (MMAD) of greater than or equal to 0.7 micron and less than 5.8 micron.

The gas mask further comprises an exhalation port that comprises a one-way valve to allow exhaled gases to be expelled from the gas mask, or the device comprises a one-way valve positioned between the branched conduit means and the secondary gas inlet, such that the one-way valve is operable to prevent the aerosol from flowing out through the secondary gas inlet.

The branched conduit means can be a conduit structure in a variety of forms. In one embodiment, the first inlet port of the branched conduit means is co-axial with the outlet port of the branched conduit means. In another embodiment, the branched conduit means comprises a T-shaped conduit connector, a Y-shaped conduit connector, or a parallel Y-shaped conduit connector.

The conduit means, gas mask, or both are in fluid communication with a reservoir bag. In one embodiment, the conduit means comprises a branched conduit which comprises a first inlet port, a second inlet port, and an outlet port, wherein the first inlet port is in communication with a discharge outlet from an aerosolization subassembly comprising the aerosolization means, the second inlet port is in communication with the reservoir bag, the outlet port is in communication with a source gas aperture in the gas mask.

## Brief Description of the Figures

FIG. 1 is a perspective view of one embodiment of the medical device described herein.

FIG. 2 is a perspective view of one embodiment of the nebulizer subassembly (top and bottom portions uncoupled) of the medical device described herein.

FIG. 3 is a perspective view of one embodiment of the nebulizer subassembly (top and bottom portions coupled) of the medical device described herein.

FIG. 4 is a perspective view of another embodiment of the medical device described herein, wherein the branched conduit means comprises a T-shaped connector.

FIG. 5 is a perspective view of another embodiment of the medical device described herein, wherein the branched conduit means comprises a parallel-Y-shaped connector.

FIG. 6 is a perspective view of another embodiment of the medical device described herein, wherein the branched conduit means comprises multiple pathways for the flow of heliox into the gas mask.

FIG. 7 is a box-and-whiskers graph of $FEV_1$ values for control and heliox groups after each of three treatments in a comparative study.

FIG. 8 is a line drawing of a particle size-testing device, which includes one embodiment of the nebulizer of the present medical device connected to a Cascade Impactor.

FIG. 9 is a line drawing of a particle size-testing device, which includes a second embodiment of the nebulizer of the present medical device connected to a Cascade Impactor.

FIG. 10 is a line drawing of a particle size-testing device that includes a prior art nebulizer connected to a Cascade Impactor.

FIG. 11 is a graph showing nebulized particle size fraction vs. aerodynamic diameter of the nebulized particles generated using one embodiment of the nebulizer of the present medical device.

FIG. 12 is a graph showing nebulized particle size fraction vs. aerodynamic diameter of the nebulized particles generated using a second embodiment of the nebulizer of the present medical device.

FIG. 13 is a graph showing nebulized particle size fraction vs. aerodynamic diameter of the nebulized particles generated using a prior art nebulizer.

FIG. 14a and 14b are front and cross-sectional views of a heliox regulator control valve for use with the present medical device.

FIG. 15 is a graph of aerodynamic diameter of particles versus mass fraction for budesonide nebulized using 18 L/min heliox flowrate to each of the nebulizer and the side port.

FIG. 16 is a graph of aerodynamic diameter of particles versus mass fraction for albuterol nebulized using 6 L/min heliox flowrate to each of the nebulizer and the side port.

FIG. 17 is a graph of aerodynamic diameter of particles versus mass fraction for albuterol nebulized using 10 L/min heliox flowrate to each of the nebulizer and the side port.

FIG. 18 is a graph of aerodynamic diameter of particles versus mass fraction for albuterol nebulized using 18 L/min heliox flowrate to the nebulizer and 0 L/min to the side port.

FIG. 19 is a graph of aerodynamic diameter of particles versus mass fraction for albuterol nebulized using 10 L/min heliox flowrate to the nebulizer and 18 L/min to the side port.

FIG. 20 is a graph of aerodynamic diameter of particles versus mass fraction for albuterol nebulized using 18 L/min heliox flowrate to the nebulizer and 10 L/min to the side port.

FIG. 21 is a perspective view of an embodiment of the medical device using a single heliox gas inlet.

FIG. 22 is a perspective view of the medical device without a reservoir bag, not according to the invention.

**Detailed Description of the Invention**

An improved medical device and method has been developed for the pulmonary administration of a drug with heliox to a patient. The device and methods are an improvement over currently available nebulizer systems in which ambient air can be introduced (e.g., via the patient's nose, open vents in the mask, etc.) and thus undesirably reduce the helium concentration over currently available nebulizer systems which use oxygen as a driving gas or that use high flow heliox in a supplemental port which does not deliver an effective amount of helium or particles in the breathable size range. The presently described device and method uses heliox as the driving gas for the nebulizer, which provides a high concentration of both helium, and drug particles in the breathable size range, and the closed system substantially avoids heliox dilution with ambient air.

As used herein, the term "closed system" in reference to the present medical device means that device with its gas mask properly secured over a patient's mouth and nose effectively has no opening operational to permit ambient air to be inhaled. In this closed system, the mask or other part of the device may, however, include openings having one-way valves to permit exhaled gases to be expelled from the system.

## The Medical Device

In a preferred embodiment, the device comprises (a) an aerosolization subassembly comprising a gas inlet for connection to a first heliox gas source; (b) a gas mask which can be secured over a patient's mouth and nose; (c) a secondary gas inlet for connection to a second heliox gas source; and (d) a branched conduit means which comprises a first inlet port, a second inlet port, and an outlet port. The aerosolization subassembly further includes (i) a drug reservoir for containing a drug to be administered, (ii) an atomization means for forming an aerosol of particles (or droplets) which comprise the drug and which are dispersed in a heliox driving gas received from the first heliox gas source, and (iii) a discharge outlet for discharging the aerosol. The gas mask comprises a source gas aperture, which is in fluid communication with the outlet port of the branched conduit means. The first inlet port of the branched conduit means is in fluid communication with the discharge outlet of the aerosolization subassembly, and the second inlet port is in fluid communication with the secondary gas inlet. The medical device is operable as a closed system to prevent dilution of the aerosol with ambient air before and during inhalation of the aerosol by the patient.

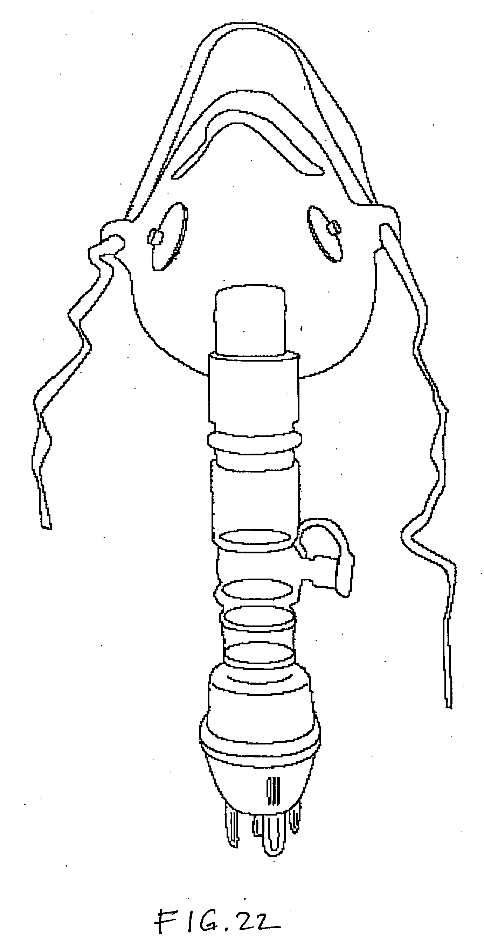

In one embodiment, the device includes a reservoir bag. For example, it can be provided in fluid communication with the second inlet port of the branched conduit means. Generally, if the total flow rate of heliox exceeds the patient's peak flow rate of breathing, then the medical device does not require the use of a reservoir bag. One embodiment of the device without the reservoir bag is shown in FIG. 22. On the other hand, it is possible for some patient's peak flow rate and peak tidal volume of breathing to exceed the total flow rate and system volume of heliox in the medical device. In that case, it is preferable for the medical device to include a reservoir bag to accommodate the peak flows and tidal volume.

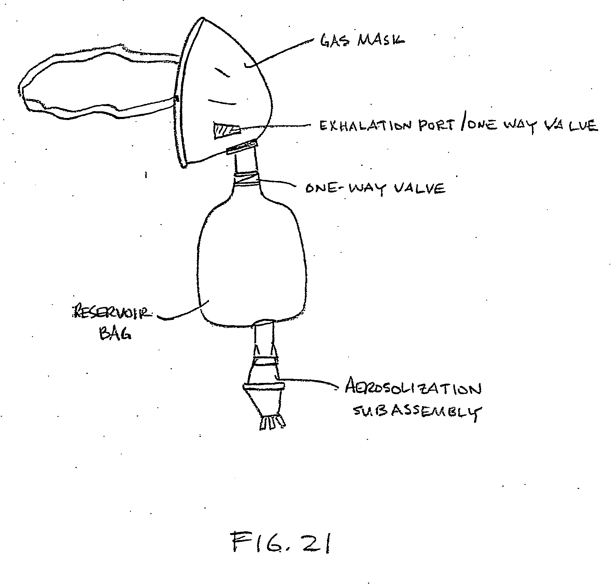

In yet another embodiment, the device includes only a single gas inlet. For example, the device could include a reservoir bag that functions like a spacer for a metered dose inhaler (MDI). That is, the aerosol is prepared and staged in the reservoir bag, for subsequent inhalation. For example, the heliox and drug could be fed into the reservoir bag, and then periodically released through a one-way valve in a connected conduit means upon demand via inhalation or mechanical ventilation. See FIG. 21.

In another embodiment, the device further includes a source of compressed heliox gas connected to the gas inlet of the nebulizer. The source of compressed heliox gas, in one embodiment, can be connected to the secondary gas inlet. In one embodiment, the source of compressed heliox gas comprises a tank coupled to a single regulator valve having two discharge outlets. In one embodiment, the single regulator valve can provide identical flow rates of heliox through each of the two discharge outlets.

The aerosolization subassembly generates a pharmaceutical aerosol. The subassembly can comprises a jet nebulizer, a pneumatic nebulizer, an ultrasonic nebulizer, or an electrostatic nebulizer, as known in the art. In a preferred embodiment, the device produces an aerosol wherein greater than 55 wt% of the drug is in the form of particles having a diameter of greater than or equal to 0.7 micron and less than 5.8 micron. In a preferred embodiment, the aerosolization subassembly comprises an AIRLIFE™ MISTY-NEB™ Nebulizer (Allegiance Healthcare, McGaw Park, Illinois, USA). Alternatively, the aerosolization subassembly can be adapted for dry powder drug delivery. That is, the subassembly can be designed to inject or release a dose of dry powder into a flowing stream of heliox gas. This could be done by adapting a known dry powder inhaler or metered dose inhaler. In one embodiment, the drug reservoir could comprise a blister or pouch containing the dose, and then the blister or pouch could be ruptured by a mechanical triggering mechanism. The gas flow then could expel the drug from the pouch or blister and disperse the drug for inhalation with the heliox.

The gas mask includes at least one aperture for receiving the drug-heliox aerosol. It optionally may include one or more exhalation ports that comprise a one-way valve to allow exhaled gases to be expelled from the gas mask. The gas mask is otherwise closed, in order to avoid entrainment of ambient air, which would undesirably dilute the helium concentration in the inhaled aerosol. In an alternative embodiment, an exhalation port is included as part of the branched conduit means, rather than in the gas mask. In such an embodiment, one or more one-way valves would be included in the branched conduit means to permit discharge of exhaled gases, while avoiding air entrainment into the system. In another embodiment, the device comprises a one-way valve positioned between the branched conduit means and the secondary gas inlet, such that the one-way valve is operable to prevent the aerosol from flowing out through the secondary gas inlet.

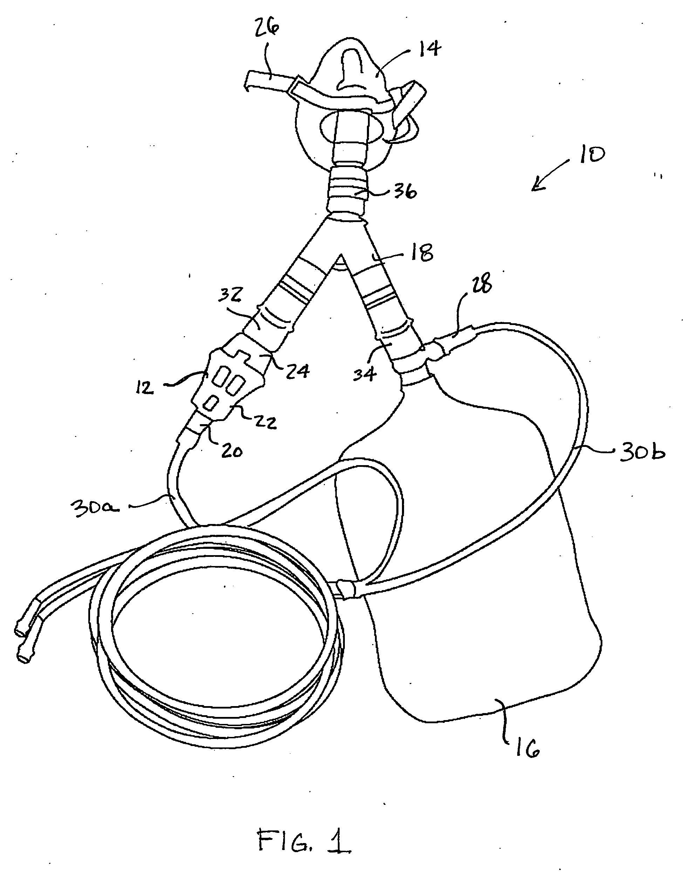

The medical device can be further understood with reference to the exemplary embodiments illustrated in FIGS. 1-3. In FIG. 1, a medical device 10 includes an aerosolization subassembly 12, a gas mask 14, a reservoir bag 16, and a branched conduit means 18. The branched conduit means 18 comprises a first inlet port 32, a second inlet port 34, and an outlet port 36. The first inlet port 32 is in communication with the discharge outlet 24 of the aerosolization subassembly 12. The second inlet port 34 is in communication with the reservoir bag 16. The outlet port 36 is connected to the source gas aperture of the gas mask 14. The gas mask 14 is provided with an elastic strap 26 for securing the mask over a patient's mouth and nose. The medical device 10 further includes a secondary gas inlet 28 for connection to a second heliox gas source. The secondary gas inlet 28 is connected to the reservoir bag 16. A pair of flexible hoses (e.g., standard or crush resistant oxygen tubing) **30a** and **30b** connect the gas inlet **20** and the secondary gas inlet **28** to the first and second heliox gas sources, respectively.

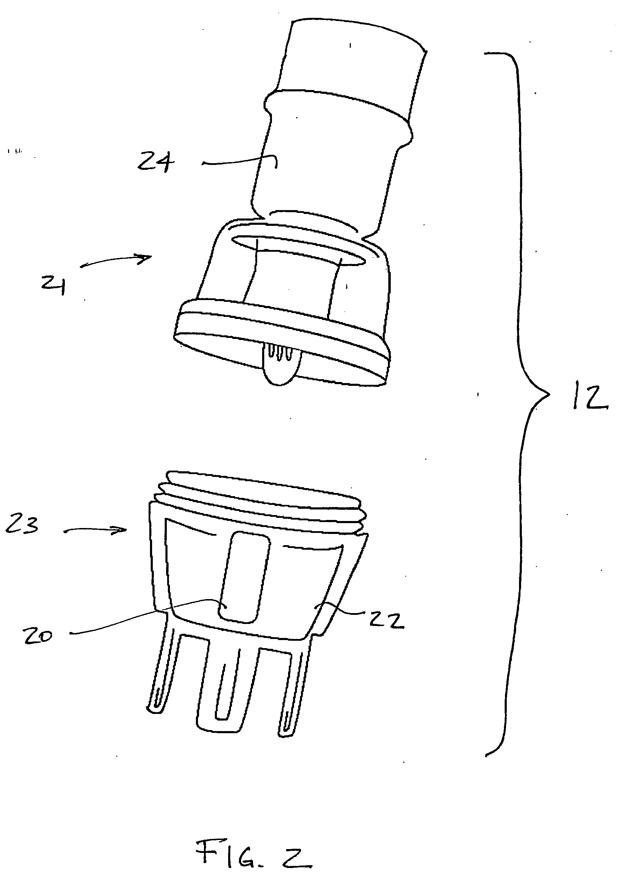

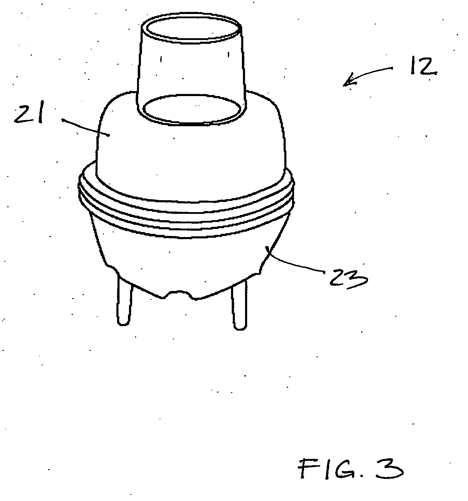

As shown in FIGS. 1-3, the aerosolization subassembly **12** includes a gas inlet **20** for connection to a first heliox gas source, a liquid reservoir **22** for containing a liquid comprising a drug, and a discharge outlet **24** for discharging the aerosol. FIG. 2 shows an aerosolization subassembly that comprises a nebulizer, with a top portion **21** uncoupled from a bottom portion **23**, for example to permit loading of the liquid reservoir with a dose of drug solution or suspension. FIG. 3 shows the top portion **21** and the bottom portion **23** coupled and ready for connection to the first heliox gas source and to the branched conduit means **18**.

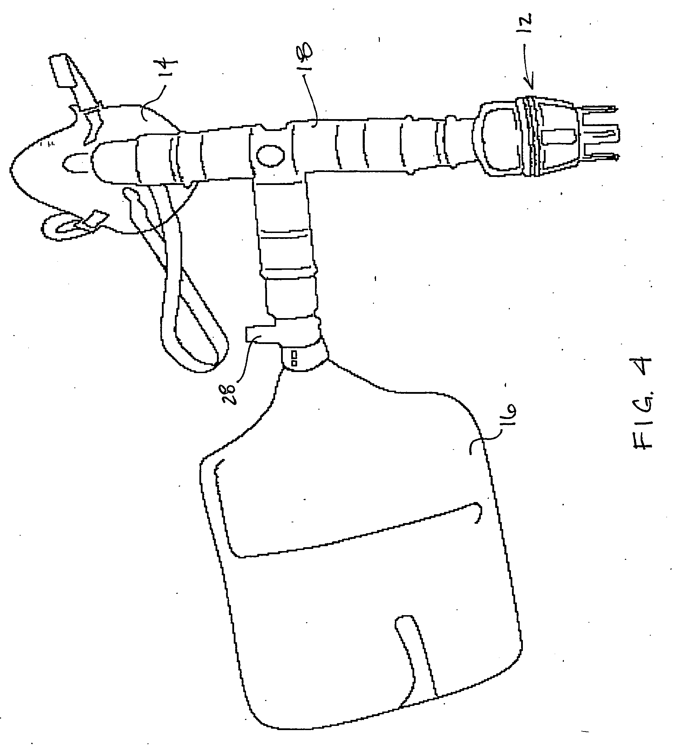

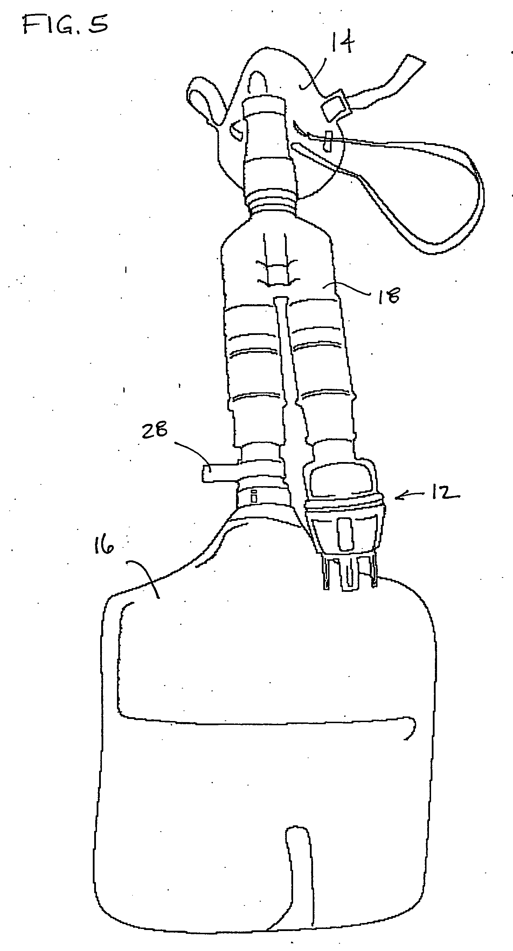

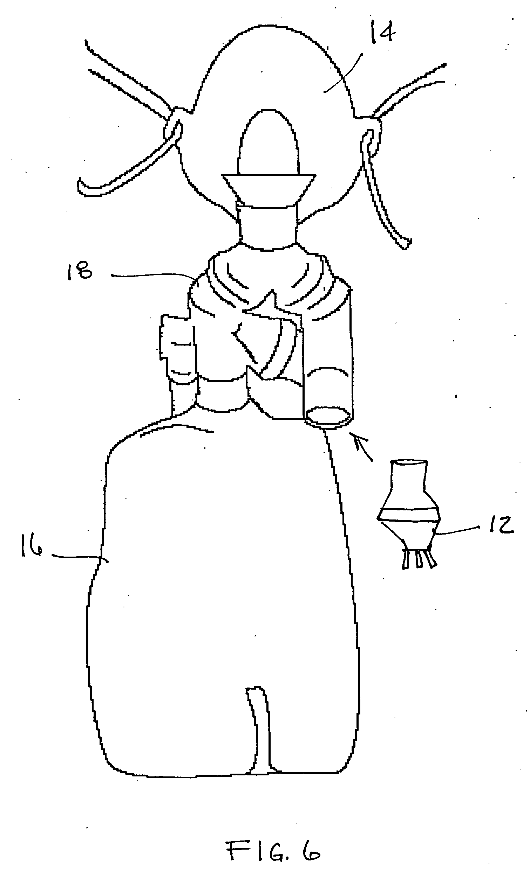

The branched conduit means is a essentially any structure that can serve as a conduit for flowing gas or aerosol. It can be formed of, e.g., assembled from, crush resistant plastic tubing and connector pieces available in the art. It can be provided in a variety of forms and designs. In one embodiment, the first inlet port of the branched conduit means is co-axial with the outlet port of the branched conduit means. In another embodiment, the branched conduit means comprises a T-shaped conduit connector, a Y-shaped conduit connector, or a parallel Y-shaped conduit connector. Several various configurations of the branched conduit means are possible. A few are illustrated in FIGS. 4-6.

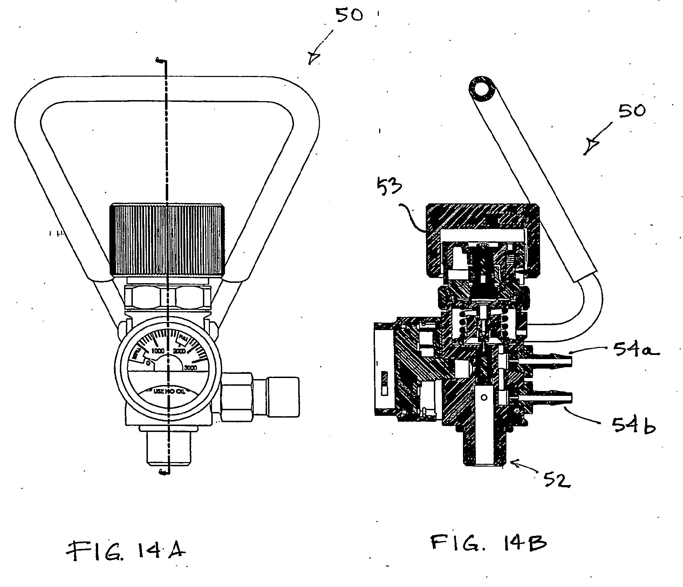

The heliox gas sources typically are one or more tanks of compressed heliox gas. In a preferred embodiment, a single tank provides both sources. Preferably, such a source tank is coupled to a single regulator valve having two gas discharge outlets for connection to the pair of flexible hoses. In one preferred embodiment, the single regulator valve provides identical flow rates of heliox through each of the two discharge outlets. Such a regulator valve is illustrated in FIGS. 14A and 14B. Valve **50** includes connector fitting **52**, for connection to a tank of compressed heliox gas. Heliox flow through discharge ports **54a** and **54b** can be controlled by adjusting the flow control knob **53**. Preferably, the heliox flow rate is set at a specified, predetermined rate and the flow control knob **53** becomes merely an "ON/OFF" knob. In an alternative embodiment, two or more control means are provided to control the flow of heliox to the gas inlet and the secondary gas inlet, e.g., with a separate control knob for controlling the gas flow rate to each inlet.

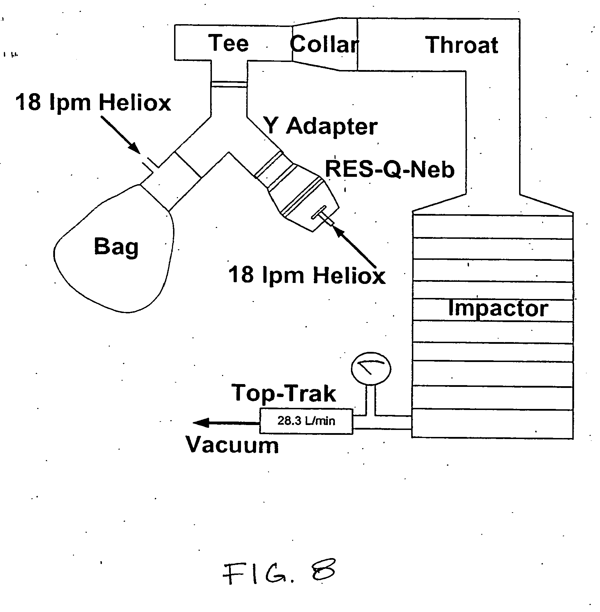

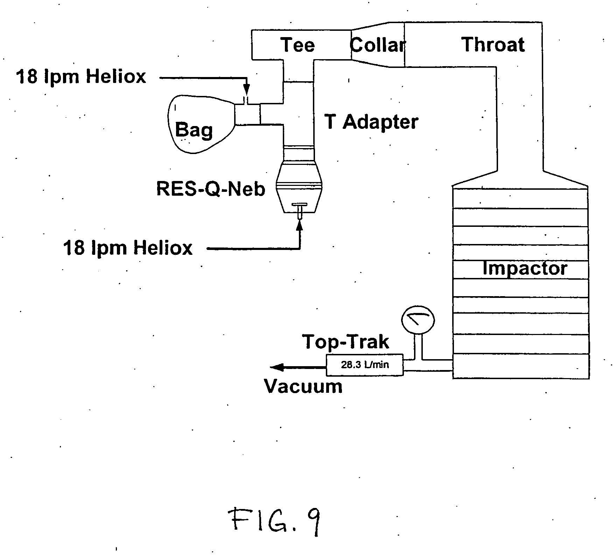

In yet another embodiment, the device is adapted for use with a breathing assistance apparatus (e.g., for mechanically ventilated patients) for use with patients unable to breath on their own.. For example, the apparatus could comprise a breathing tube for insertion into the patient's airway, which delivers heliox from a second source into the patient's lungs. In one modification, the reservoir bag could be removed and the corresponding receiver on the Y-branched conduit could be attached to the tubing coming from the ventilator (with heliox flow), and then the mask could be removed and the corresponding receiver could be attached to the tubing going to the breathing tube to allow for the system to be configured into the ventilator circuit. Alternatively, in place of a mask, the system would include a T-adapter that could connect in line with the ventilator tubing coming from the ventilator to the patient. This T-piece would be similar to the "Tee" adapter connecting the aerosolization subassembly in the FIGS. 8 and 9.

Heliox

As used herein, the term "heliox" refers to a gaseous mixture of helium and oxygen, wherein the mixture comprises greater than 50 % (vol.) helium (He) and at least 15 % (vol.) oxygen ($O_2$). In one embodiment, the heliox comprises greater than 75 % (vol.) He and between 18 and 25 % (vol.) $O_2$. In a preferred embodiment, the heliox consists essentially of about 80 % (vol.) He and 20 % (vol.)$O_2$. The heliox should meet or exceed applicable standards (e.g., for purity) set for pharmaceutical or medical gases. Such heliox is commercially available, for example, from BOC Gases (Murray Hill, New Jersey, USA).

For use with the medical device described herein, the heliox preferably is provided in a standard, pressurized, re-fillable tank. Preferably, the tank is provided with the flow control valve described herein, which has two discharge points (e.g., tubing connection barbs) for coupling to the medical device as detailed above.

Drugs

As used herein, the term "drug" includes any therapeutic, prophylactic, or diagnostic agent, which is suitable for pulmonary administration to a patient. The term "drug" includes combinations of different drugs, unless a single drug is explicitly indicated. Before aerosolization, the drug may be in a pure liquid form, in a solution comprising a pharmaceutically acceptable solvent, in a suspension of solid particles dispersed in a pharmaceutically acceptable liquid medium, or in dry powder form (pure drug or a blend of drug and one or more excipient materials known in the art). Aerosolization (e.g., by nebulization or dry powder dispersion) produces an aerosol comprising the drug. As used herein, the term "aerosol" refers to a fine dispersion of particles (e.g., liquid droplets, solid particles, or a combination thereof) dispersed in a gaseous medium, which for the present methods and devices is heliox.

In preferred embodiments, the drug is indicated for the treatment or management of respiratory diseases, such as asthma, chronic pulmonary obstructive disease (CPOD), emphysema, chronic bronchitis, bronchopulmonary dysplasia (BPD), neonatal respiratory distress syndrome (RDS), bronchiolitis, croup, post extubation stridor, pulmonary fibrosis, pneumonia, or cystic fibrosis (CF). In other preferred embodiments, the drug or combination of drugs is indicated or otherwise useful in the treatment or management of lung cancer (e.g., squamous cell carcinoma, adenocarcinoma, etc.) or AIDS. In other embodiments, the drug is for any indication where it is desirable to deliver the drug by pulmonary administration. The drug can be for systemic, regional, or local therapeutic effect or diagnostic purposes.

In one embodiment, the drug is a protein or a peptide. In another embodiment, the drug is incorporated with a monoclonal antibody.

In various embodiments, the drug is a bronchodilator, an anti-inflammatory agent, an antibiotic, an expectorant or agent effective to decrease or increase mucous production, or a combination thereof.

In a preferred embodiment, the drug comprises a bronchodilator. Representative examples of suitable types of bronchodilators include beta agonists (long acting or short acting), anticholinergics (e.g., ipratropium), and methylxanthines. Beta agonists are typically preferred. Examples of suitable beta agonists include albuterol, salbutamol, formoterol, salmeterol, pirbuterol, metaproterenol, terbutaline, and bitolterol mesylate. Albuterol, for example, is typically provided as an aqueous solution of Albuterol sulfate.

Representative examples of suitable types of anti-inflammatory agents include steroids, cromolyn, nedocromil, and leukotriene inhibitors (e.g., zafirlukast or zileuton). Corticosteriods are typically preferred steroids for inhalation. Examples of suitable corticosteroids include beclomethasone, betamethasone, ciclomethasone, dexamethasone, triamcinolone, budesonide, butixocort, ciclesonide, fluticasone, flunisolide, icomethasone, mometasone, tixocortol, and loteprednol. Preferred corticosteroids include budesonide, fluticasone propionate, beclomethasone dipropionate, mometasone, fometerol, flunisolide, and triamcinolone acetonide. Other suitable steroids for pulmonary administration include testosterone, progesterone, and estradiol.

Examples of antibiotics include penicillins (e.g., azlocillin), cephalosporins (e.g., cefotiam or ceftriaxone), carbapenems, monobatams, aminoglycosides (e.g., streptomycin, neomycin, gentamycin, amikacin or tobramycin), quinolones (e.g., ciprofloxacin), macrolides (e.g., erythromycin), nitroimidazoles (e.g., tinidazole), lincosamides (e.g., clindamycin), glycopeptides (e.g., vancomycin), and polypeptides (e.g., bacitracin). In one embodiment, the drug is tobramycin, which has been shown to be effective in managing psuedomonal infections in patients with cystic fibrosis.

In other embodiments, the drug is an antineoplastic agent, such as paclitaxel or docetaxel; a therapeutic peptide or protein, such as insulin, calcitonin, leuprolide, granulocyte colony-stimulating factor, parathyroid hormone-related peptide, or somatostatin; a monoclonal antibody; a radioactive drug; or an anti-viral agent.

Methods of Use/Treatment

The methods described hereafter do not form part of the claimed invention.

The present medical device can be used to delivery one or more drugs to a patient's lungs. The method comprises the steps of

(a) providing a medical device which comprises an aerosolization means, a conduit means, a gas mask, and at least one source of heliox gas, wherein the medical device is operable as a closed system to prevent entrainment of ambient air into an aerosol produced within said medical device; (b) providing a dose of a drug in a liquid or dry powder form; (c) using the aerosolization means to form an aerosol of particles or droplets dispersed in a first portion of heliox gas flowing at a first flow rate, wherein said particles or droplet comprise the drug and said first portion of heliox gas is from said at least one source of heliox gas; and (d) flowing the aerosol through the conduit means and into the gas mask which is secured over a patient's mouth and nose in a manner for the patient to inhale the aerosol without dilution of the aerosol with ambient air before and during inhalation of the aerosol by the patient.

The method further comprises simultaneously introducing a second heliox portion into the conduit means or into the gas mask at a second flow rate. In this embodiment, the first and second heliox portions together preferably comprise between about 50 and 85 vol.% helium (e.g., between about 60 and 85 vol.% helium, between 70 and 80 vol.% helium, or about 80 vol.% helium). In one case, the first and second heliox portions each comprise 80 vol.% helium.

Alternatively the first flow rate is between 6 and 30 liters per minute, preferably between 15 and 20 liters per minute. In the case where there is a second heliox portion introduced simultaneously into the conduit means or into the gas mask at a second flow rate, the second flow rate preferably is between 6 and 30 liters per minute, more preferably between 15 and 20 liters per minute. In one case, the first flow rate and the second flow rate each are about 18 liters per minute. In another case, the combined flow rate of the first and second heliox portions provided to the medical device is between 6 and 60 liters per minute (e.g., between 25 and 45 liters per minute, between 30 and 40 liters per minute, or about 36 liters per minute). In one embodiment using a jet or pneumatic nebulizer, the first and second heliox portions together comprise 80 vol.% helium, and the combined flow rate of the first and second heliox portions provided to the medical device is between 30 and 40 liters per minute.

As an alternative the amount of drug provided and the manner of operation of the aerosolization means are effective to delivery a dose of drug over a period between 1 and 30 minutes (e.g., between about 5 and 15 minutes).

Generally, a dose of drug is first loaded into the aerosolization subassembly, and the aerosolization assembly is connected, if not already connected, to the branched conduit. Heliox supply lines are connected to the aerosolization subassembly and to the secondary gas inlet. Then, the flow of heliox to the device is begun to both gas inlets of the device.

The flow rate of heliox to the aerosolization subassembly preferably is between 5 and 25 L/min, more preferably between 15 and 20 L/min, and most preferably 18 L/min. The flow rate of heliox to the secondary gas inlet preferably is up to a combined total of 70 L/min (e.g., more than 10, 15, 20, or 30 L/min, and e.g., less than 60, 50, 40, or 35 L/min).

For the treatment of a moderate or severe acute asthma exacerbation or other reversible upper respiratory obstruction, the aerosolization subassembly comprises a nebulizer, preferably an AIRLIFE™ MISTY-NEB™ Nebulizer, the flow rate of heliox to the nebulizer is between 15 and 20 L/min (e.g., 18 L/min), the drug is a bronchodilator (e.g., a beta-agonist, such as albuterol), and the flow rate of heliox to the reservoir bag (from the secondary gas inlet) is between 15 and 20 L/min (e.g., 18 L/min).

The actual heliox flow rates for a particular application should be selected to maximize the respirable fraction of the drug to be delivered, e.g., to achieve a high mass % of drugs which in the aerosol form are comprised in particles having an aerodynamic diameter between about 1 and 5 microns, and preferably normally distributed around about 1 to 1.5 micron, over the desired delivery period. Heliox flow rates can be critical, as the use of heliox to produce aerosolized drug changes the particle size distribution and efficiency of the nebulizer due to the heliox gas density difference as compared to currently used gases (e.g., compressed oxygen or compressed air). For example, at an equivalent flow rates to oxygen or compressed air, aerosolization with heliox results in a different particle size distribution and less total output delivered from the nebulizer. By adjusting the flow rates (typically by increasing the flow rate) of heliox, the particle size distribution and total output can be optimized to be equivalent to or better than those nebulizers driven with oxygen or compressed air. These factors, coupled with the ability of the heliox gas to convert turbulent areas of flow to laminar flow, maximize drug penetration and distribution in the lungs. The aerosol comprises at least about 60% helium in order to convert turbulent flow to laminar flow in patients.

The drug delivery methods described herein are useful in the treatment or management of respiratory diseases including, but not limited to, asthma, chronic pulmonary obstructive disease (CPOD), emphysema, chronic bronchitis, bronchopulmonary dysplasia (BPD), neonatal respiratory distress syndrome (RDS), bronchiolitis, croup, post extubation stridor, pulmonary fibrosis, cystic fibrosis (CF), bacterial or viral infections, tumor growth, cancer, pneumonia, and/or lung tissue damage due to burns or smoke inhalation.

A method of treating a patient suffering from acute partial upper airway obstruction and/or inflammation comprises the steps of (a) providing a medical device which comprises an aerosolization means, a conduit means, a gas mask, and at least one source of heliox gas, wherein the medical device is operable as a closed system to prevent entrainment of ambient air into an aerosol produced within said medical device; (b) providing a dose of a drug in a liquid or dry powder form, said drug including a bronchodilator, an anti-inflammatory agent, or both; (c) using the aerosolization means of the medical device to form an aerosol of particles or droplets dispersed in heliox gas from said at least one source of heliox, wherein said particles or droplet comprise said drug; and (d) flowing the aerosol through the conduit means and into the gas mask which is secured over a patient's mouth and nose in a manner for the patient to inhale the aerosol. Preferably, the helium concentration in the aerosol inhaled by the patient is between 60 and 85 vol.% (e.g., between about 70 and 80 vol.%).

In one example, the bronchodilator comprises an alpha agonist, a beta agonist, or a racemic mixture thereof. Examples of beta agonists included albuterol, formoterol, salmeterol, pirbuterol, metaproterenol, terbutaline, and bitolterol mesylate. In one case, the bronchodilator comprises a racemic mixture of epinephrine, which exhibits an alpha and beta agonist activity.

In another example, the bronchodilator comprises an anticholinergic, such as one that comprises ipratropium bromide.

In yet another example, the anti-inflammatory agent is selected from steroids, cromolyn, nedocromil, and leukotriene inhibitors. In one case, the steroid is a corticosteroid. Examples of corticosteroid beclomethasone, betamethasone, ciclomethasone, dexamethasone, triamcinolone, budesonide, butixocort, ciclesonide, fluticasone, flunisolide, icomethasone, mometasone, tixocortol, loteprednol, budesonide, fluticasone propionate, beclomethasone dipropionate, mometasone, fometerol, flunisolide, triamcinolone acetonide, testosterone, progesterone, and estradiol.

The present invention can be best understood with reference to the following non-limiting examples.

**Example 1: Albuterol-Heliox Treatment of Asthma-Prior Art**

A pilot study was conducted using an open system, including a mouthpiece and T-piece adapter (Airlife™ Misty-Neb™, Allegiance Healthcare Corp., McGaw Park, IL), to nebulize albuterol. In this pilot study, control patients had received albuterol nebulized with oxygen, while study patients received albuterol nebulized with heliox through this "open" mouthpiece and T-piece adapter breathing system. From this study, no differences in spirometry between heliox and oxygen albuterol nebulization were detected.

It was hypothesized that entrainment of ambient air was decreasing the effective concentration of heliox being delivered to the patient with this system.

**Example 2: Albuterol-Heliox Treatment of Asthma Without Dilution of Heliox by Entrained Air**

In view of the pilot study and hypothesis on air entrainment described in Example 1, a new delivery system (an earlier prototype of the device shown in FIG. 1) was designed to assure delivery of a high concentration of heliox to a patient's airways. A new study was conducted to test the hypothesis.

Forty-five patients were randomized to receive albuterol nebulized with oxygen (control) versus heliox. At baseline, demographics, outpatient asthma medications, vital signs, oxygen saturation and forced expiratory volume in one second ($FEV_1$) were not different between the two groups. Three consecutive albuterol treatments were given to each group.

Study Subjects

The study subjects were adult patients who presented to the Emergency Department at the University of Chicago Medical Center for a severe acute exacerbation of asthma. The study was approved by the Institutional Review Board and all patients enrolled gave written informed consent before beginning the study. Patients 50 years of age or under meeting American Thoracic Society criteria for the diagnosis of asthma were eligible. To assure that only those with severe persistent asthma were studied, only those patients with baseline forced expiratory volume in one second ($FEV_1$) less than fifty percent predicted were enrolled in the study.

Study Design and Treatment Protocol

Patients were randomized to receive albuterol nebulized with either oxygen (control) or heliox 80:20 as the driving gas. The heliox delivery system consisted of a facemask connected to a Y-piece with the nebulizer (Airlife™ Misty-Neb™ Nebulizer) on one limb and a non-rebreather bag on the other limb (as shown in FIG. 1). Heliox flow to the nebulizer was set at 10 liters/minute (via an oxygen calibrated flow meter thus equivalent to 18 L/min Heliox flow). A separate flow of 10 liters/minute of heliox (via an oxygen calibrated flow meter thus equivalent to 18 L/min Heliox flow) was delivered to the non-rebreather bag. Control patients received oxygen-nebulized albuterol (at 10 L/min) delivered through this identical semi-closed breathing system. Patients in both heliox and control groups were given a total of three consecutive albuterol treatments, each consisting of 0.5 ml of 0.5% albuterol mixed in 2.5 ml of 0.9% saline. Each treatment continued until the nebulizer was dry-a period of approximately ten minutes, followed by a fifteen minute washout period. The total time for the study was approximately 90 minutes. Corticosteroid therapy for asthma exacerbations was directed by the emergency room physicians, who were not directly involved in the study.

Spirometric Measurements

Forced expiratory volume in one second ($FEV_1$) was measured at baseline and 15 minutes after completion of each albuterol treatment using a portable Vacumed Micro Spirometer (Ventura, CA). To assure its accuracy for the purpose of this study, the spirometer was tested every other week using a three liter syringe. In addition, the accuracy of $FEV_1$ recordings was tested by having subjects perform forced expiratory maneuvers through the portable spirometer at varying flow rates while it was in-line with a Medical Graphics 1070 pulmonary function testing system. (This system was calibrated daily.) This test was also performed every other week for the first three months of the study and monthly thereafter.

Patients performed a minimum of three forced expiratory maneuvers with values recorded according to the American Thoracic Society recommendations (American Thoracic Society: Standardization of spirometry-1994 update, Am. J. Respir. Crit. Care Med 152:1107-36 (1995)). All spirometric measurements were made by investigators who were blinded to the gas used for albuterol nebulization. For investigator blinding purposes, the study investigator was not present during the albuterol treatments and did not enter the patient's room until 15 minutes after the treatment was finished. Patients were not told explicitly which treatment limb they were randomized to; however, because of potential voice changes while breathing heliox, it was not possible to assure that patients were blinded to which therapy they received.

Statistical Analysis

Demographic data, chronic asthma medications, emergency room corticosteroid administration, vital signs (heart rate, mean arterial pressure, respiratory rate), oxygen saturation by pulse oximetry, emergency room length of stay and hospital admissions from the emergency room were recorded for all patients. Data are expressed as mean $\pm$

SD when normally distributed and median (interquartile range) when not normally distributed. Interval data were compared using a two-tailed Student's t test or Mann Whitney U test according to normality of distribution, categorical data were compared using Chi square or Fisher Exact test when appropriate. Percent changes in vital sign measurements from baseline ([vital sign post albuterol treatment - baseline vital sign/baseline vital sign] x 100) after each nebulizer treatment were compared using two-way repeated measures analysis of variance (ANOVA). The Student-Newman-Keuls test was used to compare differences between heliox and control groups after each nebulization treatment when appropriate. Raw $FEV_1$ values and percent change in $FEV_1$ from baseline ([$FEV_1$ post albuterol treatment - baseline $FEV_1$/baseline $FEV_1$] x 100) for the two groups were compared using the Mann-Whitney U test with Bonferroni's correction for multiple comparisons. A 25% difference in percent change in $FEV_1$ between the control and heliox groups was considered clinically important. Using a standard deviation of 28%

(based on preliminary work), an $\alpha$ error of 0.05 and a $\beta$ error of 0.20, it was estimated that a total of 42 patients (21 in each group) would be needed to avoid a type II error. A P value of < 0.05 was considered to indicate statistical significance, except when Bonferroni's correction for comparisons of three consecutive albuterol treatments was applied. In this situation, a P value of < 0.0167 was considered to indicate statistical significance.

Results

A total of 45 (16 men, 29 women) patients were enrolled in this study. As seen in Table 1, there were no demographic differences between the two groups. Twenty-two patients were randomized to albuterol nebulized with oxygen (control) and 23 were randomized to albuterol nebulized with heliox. Heart rate, mean arterial blood pressure, respiratory rate and oxygen saturation did not differ between the two groups at baseline (Table 2). However, as outlined in Table 2, the percent change in heart rate after albuterol nebulization was significantly different in the heliox group when compared with the control group. Overall, the heliox group demonstrated an increase in heart rate from baseline, compared to a decrease in heart rate in the control group. There were no other significant differences in any vital signs or in pulse oximetry between the groups at any point during the study (Table 2).

Baseline $FEV_1$ values (both raw and percent predicted) were not different between the two groups (Table 1). Hospital admission rates were similar in both groups (6 of 23 in the heliox group and 6 of 22 in the control group, P = 0.81). There were no differences between the two groups with regard to outpatient use of $\beta_2$ agonists, inhaled corticosteroids, systemic corticosteroids, theophylline or leukotriene inhibitors (Table 1). Tobacco use was rare and there were no differences in smoking habits between the two groups (Table 1). Nineteen of 23 in the heliox group and 20 of 22 in the control group received systemic corticosteroid treatment in the emergency room (P = 0.67).

The accuracy of the portable spirometer remained within American Thoracic Society guidelines for monitoring devices throughout the study. During each check of its calibration, $FEV_1$ values were always within 5% of the value obtained simultaneously from the spirometer in the pulmonary function lab. Similar satisfactory results were noted upon testing the portable spirometer against the three liter syringe. All patients enrolled in the study were able to perform three forced expiratory maneuvers according to American Thoracic Society recommendations (American Thoracic Society: Standardization of spirometry-1994 update, Am. J Respir. Crit. Care Med. 152:1107-36 (1995)).

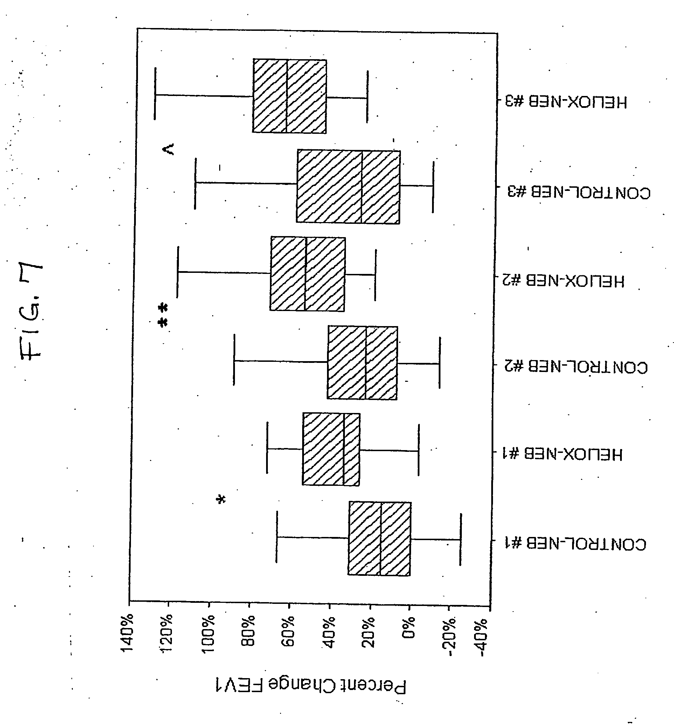

After each of the three albuterol treatments, the heliox group had a higher percent change in $FEV_1$ than the control group. Following albuterol treatment 1, median percent change in $FEV_1$ was 14.6% in the control group and 32.4% in the heliox group (P = 0.007). After treatment 2, the results were 22.7% vs. 51.5% respectively (P = 0.007). After treatment 3, the results were 26.6% vs. 65.1% respectively (P = 0.016). Using Bonferroni's correction for multiple comparisons, the difference was significant after each nebulizer treatment. These results are detailed graphically in FIG. 7, which shows $FEV_1$ values for control and heliox groups after each of three treatments. Solid horizontal line within the box is the median value. Box delineates interquartile range. Whiskers are lines that extend from the box to the highest and lowest values, excluding outliers. (* p = 0.007 for comparison between control and heliox $FEV_1$ after first albuterol nebulizer treatment. ** p = 0.007 for comparison between control and heliox $FEV_1$ after second albuterol nebulizer treatment

^ p = 0.016 for comparison between control and heliox $FEV_1$ after third albuterol nebulizer treatment).

The percent change in $FEV_1$ in the heliox group was significantly greater than control in patients with similar degrees of airflow obstruction at baseline. The patients had severe airflow obstruction based on their baseline percent predicted $FEV_1$ values. In the heliox group, the percent change in $FEV_1$ was more than twice that of the control group at all three points studied. At each post-treatment spirometric measurement, the difference was significant by Mann-Whitney U testing and remained so after Bonferroni correction.

Discussion

This study shows that albuterol nebulized with heliox leads to more effective bronchodilation when compared to albuterol nebulized with oxygen in patients presenting to the emergency room with severe acute asthma exacerbations.

It suggests that heliox is more efficient than oxygen as a vehicle for jet nebulization of albuterol during asthma exacerbations, as manifest by improvements in spirometry. Therefore, helium, when inhaled in a sufficiently high concentration, can deliver nebulized albuterol to its site of action more efficiently than oxygen in patients with acute asthma exacerbations. The difference in heart rate responses in the two groups, likely the result of more effective albuterol delivery to distal airways in the heliox group, supports this supposition.

### Table 1: Patient Summary

|  | Control | Heliox | P value |
|---|---|---|---|
| N | 22 | 23 | |
| Age | 34.7 ± 10.9 | 30.3 ± 8.6 | 0.14 |
| # Male/Female | 9/13 | 7/16 | 0.67 |
| Height (cm) | 171.2 ± 10.2 | 167.8 ± 12.3 | 0.33 |
| Baseline $FEV_1$ (liters) | 1.18 ± 0.38 | 1.17 ± 0.40 | 0.93 |
| Baseline $FEV_1$ (% predicted) | 32.8 ± 11.3 | 32.4 ± 9.2 | 0.91 |
| Chronic Asthma Medications | | | |
| β-2 agonist (%) | 17 (77%) | 20 (87%) | 0.46 |
| Inhaled corticosteroids (%) | 9 (41%) | 6 (26%) | 0.35 |
| Systemic corticosteroids (%) | 5 (23%) | 1 (4%) | 0.10 |
| Theophylline (%) | 4 (18%) | 1 (4%) | 0.19 |
| Leukotriene inhibitors (%) | 3 (14%) | 1 (4%) | 0.35 |
| Tobacco use (%) | 2 (9%) | 1 (4%) | 0.61 |
| Age, Height and Baseline $FEV_1$ (liter and % predicted) reported as mean ± SD | | | |

### Table 2: Vital Signs and Pulse Oximetry

|  | Control | Heliox | P value |
|---|---|---|---|
| Number of Test Subjects | 22 | 23 | |
| Baseline HR (beats/minute) | 103.6 ± 21.2 | 96.2 ± 18.2 | 0.22 |
| % change HR by RM ANOVA | | | 0.009 |
| % change HR post neb 1 (S-N-K) | -8.7 ± 7.4 | -0.9 ± 7.8 | 0.01 |
| % change HR post neb 2 (S-N-K) | -4.6 ± 8.7 | 1.0 ± 11.3 | 0.08 |
| % change HR post neb 3 (S-N-K) | -2.7 ± 12.7 | 5.6 ± 12.0 | 0.01 |
| Baseline MAP (mm Hg) | 92.7 ± 12.3 | 90.9 ± 14.4 | 0.64 |
| % change MAP by RM ANOVA | | | 0.33 |
| Baseline RR (breaths/minute) | 22.4 ± 4.5 | 20.7 ± 6.3 | 0.29 |
| % change RR by RM ANOVA | | | 0.78 |
| Baseline $SpO_2$ (%) | 95.9 ± 2.6 | 95.1 ± 2.2 | 0.29 |
| % change $SpO_2$ by RM ANOVA | | | 0.51 |
| HR = heart rate, RM ANOVA = two-way repeated measures analysis of variance, S-N-K = Student Newman Keuls, MAP = mean arterial pressure, RR = respiratory rate, $SpO_2$ = oxygen saturation by pulse oximetry, NS = not significant. All values reported as mean ± SD | | | |

Intuitively, it would seem that, for some patients, the impact of standard therapy (inhaled $\beta_2$ agonists and systemic corticosteroids) would largely outweigh any additional benefit derived from heliox. This speculation is likely to apply whether heliox is used to decrease respiratory work based on its physical gas properties or its activity as a gas to nebulize albuterol to its site of action. From a practical standpoint, nebulizing albuterol with heliox serves two purposes-improved airflow based on the physical properties of the gas, as well as improved spirometry presumably from improved delivery of albuterol to its site of action in the lungs.

This study shows that jet nebulization of albuterol with heliox improves spirometric measurements in patients with acute asthma more than standard oxygen driven albuterol nebulization, and that heliox should be considered as a vehicle for albuterol nebulization in patients presenting with severe acute asthma exacerbations. All asthmatics who are given heliox to breathe for its physical gas properties should receive this treatment using a high flow, non-rebreathing closed delivery system. In addition, asthmatic patients already receiving heliox should have albuterol treatments nebulized with heliox as the gas flowing into the nebulizer.

### Example 3: Nebulized Particle Size Distribution-Present Heliox Nebulization System vs. Prior Art Air Nebulization System

A study was conducted to compare the performance of two embodiments of the heliox nebulization system described herein (called the RES-Q-Neb™ Nebulizer) with a commercially available nebulizer (HOPE™, B&B Medical Technologies, Inc., Orangevale, California, USA) that is driven by air. Albuterol sulfate was the drug nebulized.

Measurements were done using an Anderson Cascade Impactor (ACI) operated at an air flow rate of 28.3 L/min. The nebulizers were connected one at a time to the same tee piece and collar. The ACI consisted of eight stages, a backup filter, and an inlet (USP throat). Cut-size was as shown in Table 3.

#### Table 3: ACI Operation Parameters

| Nebulizer System Part | Cut-Size (micron) |
| --- | --- |
| USP Throat | >9 |
| Stage 0 | 9 |
| Stage 1 | 5.8 |
| Stage 2 | 4.7 |
| Stage 3 | 3.3 |
| Stage 4 | 2.1 |
| Stage 5 | 1.1 |
| Stage 6 | 0.7 |
| Stage 7 | 0.4 |
| Back-up Filter | < 0.4 |

Gas flows through the nebulizers and impactor were controlled with rotameters, which were set with the DryCal Flow Calubrator (BIOS International Co., Pomton Plains, NJ).

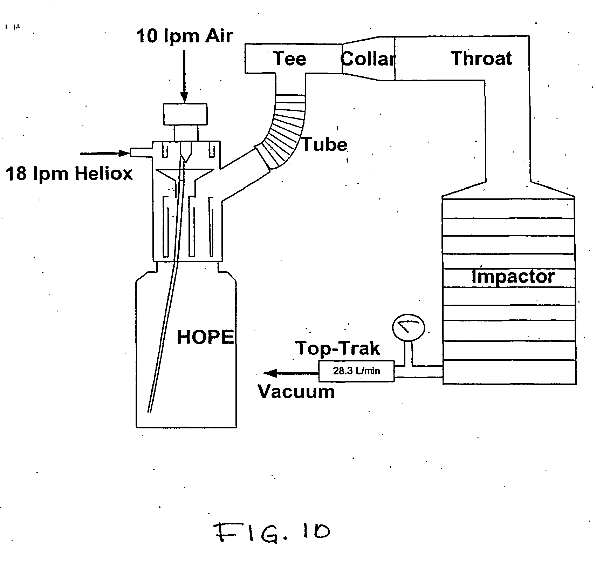

Heliox (80% helium, 20% oxygen) at 18 L/min was used to drive the RES-Q-Neb Nebulizer and another 18 L/min of the heliox was introduced into the system at the reservoir bag side port. In one embodiment, the RES-Q-Neb Nebulizer included a "Y" adaptor (a branched conduit means) as shown in FIG. 8. In another embodiment, the RES-Q-Neb included a "T" adaptor (another branched conduit means) as shown in FIG. 9. The choice of adaptor affected the angular position of the nebulizer, which in turn affected total output from the nebulizer and how long the nebulizer was able to operate before sputtering. The "Y" adaptor places the nebulizer at a 45° angle, and with only 3 mL of the drug in the reservoir, the nebulizer runs for about one minute before sputtering; however, if 5 mL (the maximum amount) of the drug is placed in the reservoir, then the liquid level exceeds the maximum line on one side of the nebulizer, but takes about 4 minutes of operation before sputtering begins. In contrast, the "T" adaptor places the nebulizer in a vertical position, and the nebulizer can run for about four minutes when 5 mL of the drug is loaded into the reservoir. Notably, the difference in the position of the nebulizer does not affect the aerosol generation rate. The HOPE nebulizer was driven with compressed air at 10 L/min (as directed in its included label and instructions), and 18 L/min of heliox was introduced into the system at a side port, as shown in FIG. 10.

A pre-running time was used for each test to stabilize the generation of aerosol. The RES-Q-Neb nebulizer was pre-run for 15 s, and the HOPE nebulizer was pre-run for 30 s. Six tests were run with the RES-Q-Neb nebulizer at the same conditions (3 with the "Y" adaptor and 3 with the "T" adaptor) and five tests were run with the HOPE nebulizer at the same conditions.

After each test, the ACI and empty nebulizers were disassembled and washed with deionized water. The wash water was collected and the amount of drug remaining or collected was determined using spectrophotometric analysis at 224 nm. Absorbance measurements were converted into Albuterol concentration values base on a calibration curve. From known concentration values and wash volumes, the mass of albuterol sulfate collected at each ACI stage was calculated.

The particle sizes are expressed as 50% mass median aerodynamic diameters (MMAD).

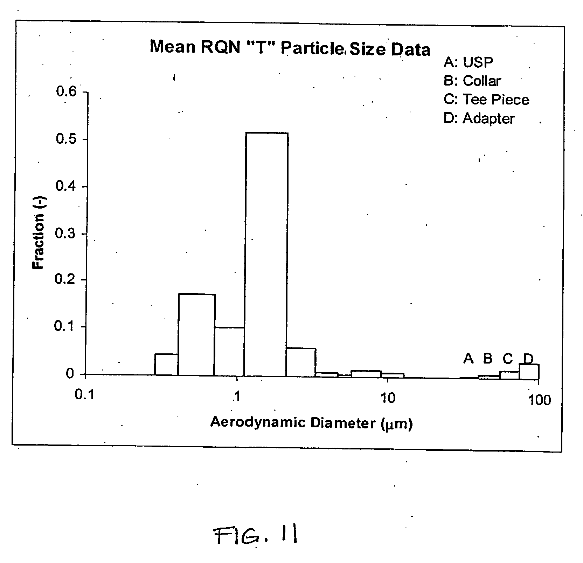

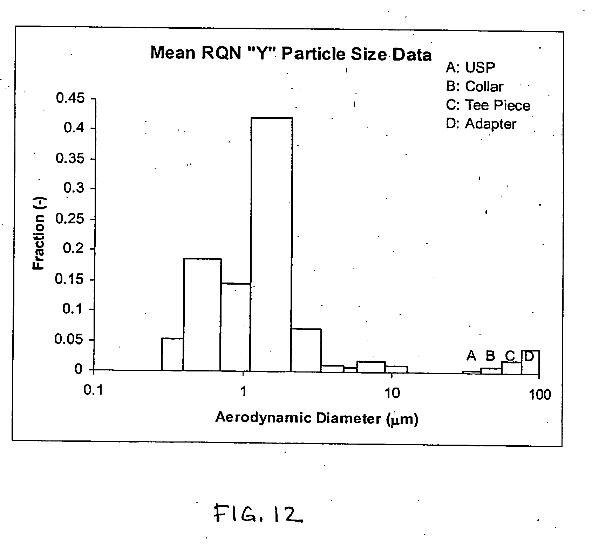

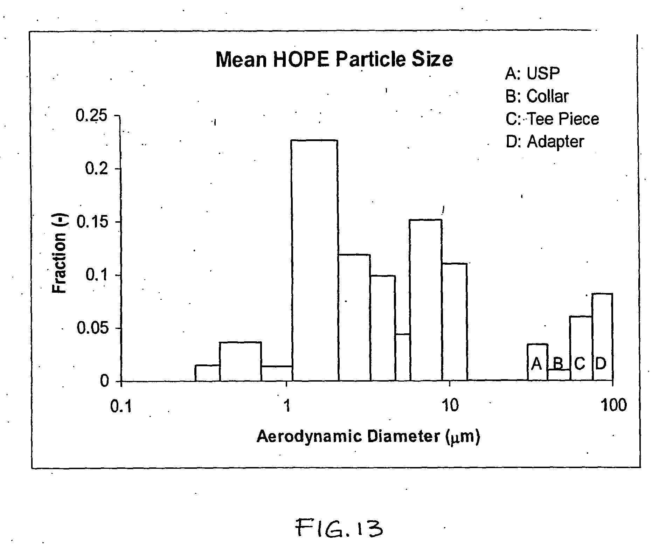

The particle size distribution for the mean of each nebulizer system is shown in Tables 4-6 and in FIGS. 11-13. There is no significant difference between the "Y" and the "T" adaptor embodiments of the RES-Q-Neb. The HOPE Nebulizer shows two peaks for the distribution, whereas the RES-Q-Neb nebulizer shows a single peak. The mean output rates of the Y and T designs were similar (0.59 mL/min vs. 0.54 mL/min, respectively).

**Table 4: Results Using RES-Q-Neb ("T" Design) Nebulizer**

| Stage | 1B Fraction | 2A Fraction | 1C Fraction | Sum | Mean |
|-------|-------------|-------------|-------------|-------|-------|
| Adapter | 0.023 | 0.020 | 0.055 | 0.098 | 0.033 |
| T-Piece | 0.017 | 0.024 | 0.010 | 0.051 | 0.017 |
| Collar | 0.010 | 0.010 | 0.002 | 0.023 | 0.008 |
| Throat | 0.000 | 0.009 | 0.000 | 0.010 | 0.003 |
| 0 | 0.007 | 0.018 | 0.005 | 0.030 | 0.010 |
| 1 | 0.011 | 0.026 | 0.008 | 0.044 | 0.015 |
| 2 | 0.003 | 0.011 | 0.001 | 0.015 | 0.005 |
| 3 | 0.005 | 0.017 | 0.005 | 0.027 | 0.009 |
| 4 | 0.044 | 0.094 | 0.047 | 0.184 | 0.061 |
| 5 | 0.688 | 0.429 | 0.440 | 1.557 | 0.519 |
| 6 | 0.006 | 0.127 | 0.176 | 0.309 | 0.103 |
| 7 | 0.148 | 0.166 | 0.207 | 0.521 | 0.174 |
| Filter | 0.037 | 0.049 | 0.045 | 0.131 | 0.044 |
| Total | 1.000 | 1.000 | 1.000 | 3.000 | 1.000 |

**Table 5: Results Using RES-Q-Neb ("Y" Design) Nebulizer**

| Stage | 1B Fraction | 2A Fraction | 1C Fraction | Sum | Mean |
|-------|-------------|-------------|-------------|-------|-------|
| Adapter | 0.038 | 0.069 | 0.014 | 0.121 | 0.040 |
| Y- Piece | 0.026 | 0.022 | 0.015 | 0.062 | 0.021 |
| Collar | 0.004 | 0.013 | 0.010 | 0.028 | 0.009 |
| Throat | 0.000 | 0.011 | 0.001 | 0.012 | 0.004 |
| 0 | 0.005 | 0.022 | 0.009 | 0.035 | 0.012 |
| 1 | 0.014 | 0.023 | 0.020 | 0.056 | 0.019 |
| 2 | 0.000 | 0.016 | 0.003 | 0.020 | 0.007 |
| 3 | 0.005 | 0.018 | 0.007 | 0.030 | 0.010 |
| 4 | 0.067 | 0.068 | 0.080 | 0.215 | 0.072 |
| 5 | 0.467 | 0.355 | 0.443 | 1.265 | 0.422 |
| 6 | 0.148 | 0.144 | 0.148 | 0.439 | 0.146 |

(continued)

| Stage | 1B Fraction | 2A Fraction | 1C Fraction | Sum | Mean |
|---|---|---|---|---|---|
| 7 | 0.169 | 0.180 | 0.208 | 0.557 | 0.186 |
| Filter | 0.056 | 0.060 | 0.043 | 0.159 | 0.053 |
| Total | 1.000 | 1.000 | 1.000 | 3.000 | 1.000 |

**Table 6: Results Using HOPE Nebulizer**

| Stage | 1B Fraction | 2A Fraction | 1C Fraction | 1D Fraction | 2B Fraction | Sum | Mean |
|---|---|---|---|---|---|---|---|
| Tube | 0.105 | 0.077 | 0.073 | 0.081 | 0.074 | 0.409 | 0.082 |
| Tee Piece | 0.066 | 0.056 | 0.058 | 0.052 | 0.069 | 0.301 | 0.060 |
| Collar | 0.016 | 0.004 | 0.008 | 0.014 | 0.006 | 0.047 | 0.009 |
| Throat | 0.033 | 0.037 | 0.036 | 0.030 | 0.035 | 0.171 | 0.034 |
| 0 | 0.098 | 0.123 | 0.116 | 0.096 | 0.117 | 0.550 | 0.110 |
| 1 | 0.139 | 0.164 | 0.154 | 0.138 | 0.160 | 0.755 | 0.151 |
| 2 | 0.039 | 0.047 | 0.044 | 0.045 | 0.044 | 0.218 | 0.044 |
| 3 | 0.088 | 0.110 | 0.109 | 0.090 | 0.099 | 0.496 | 0.099 |
| 4 | 0.118 | 0.112 | 0.120 | 0.121 | 0.123 | 0.595 | 0.119 |
| 5 | 0.221 | 0.205 | 0.238 | 0.234 | 0.235 | 1.133 | 0.227 |
| 6 | 0.017 | 0.017 | 0.005 | 0.026 | 0.003 | 0.068 | 0.014 |
| 7 | 0.038 | 0.035 | 0.033 | 0.052 | 0.025 | 0.184 | 0.037 |
| Filter | 0.023 | 0.012 | 0.007 | 0.022 | 0.010 | 0.074 | 0.015 |
| Total | 1.000 | 1.000 | 1.000 | 1.000 | 1.000 | 5.000 | 1.000 |

Those skilled in the art are aware of critical particle size fractions that are indicative and important parameters for identifying particles capable of being inhaled and deposited. For example, particles in the breathable range are most often described as those sized between 1 and 5 $\mu$m, as particles above 5 $\mu$m tend to be deposited or filtered in the mouth, nose, and throat, while those less than 0.5 tend to be inhaled and exhaled without being deposited. The preferred aerosol generation devices are those that produce particles having a size most closely distributed to between 1 and 1.5 $\mu$m, as this size tends to maximize alveolar deposition. Based on this knowledge, the data clearly demonstrate the significant improvement of the RES-Q-Neb-comprising medical device of the present invention over the prior art HOPE nebulizer, which demonstrate that the present devices provide a preferable heliox aerosolization system.

Table 7, for example, illustrates the significant difference between some of the different embodiments of the present devices and those of the HOPE nebulizer as depicted by mean fraction of particles within and outside of a size range generally considered to indicate suitability for pulmonary deposition. The ACI test method parameters resulted in cut offs slightly different than 1 $\mu$m and 5 $\mu$m, were 0.7 $\mu$m and 5.8 $\mu$m, respectively, and therefore were substituted for classification purposes.

**Table 7: Comparison of Critical Particle Size Fractions**

| 0.7 ≤ Mean Fraction < 5.8 $\mu$m | | |
|---|---|---|
| "T" Design | "Y" Design | HOPE |
| 0.697 | 0.657 | 0.503 |

(continued)

| Mean Fraction ≥ 5.8 μm | | |
|---|---|---|
| "T" Design | "Y" Design | HOPE |
| 0.086 | 0.105 | 0.446 |
| Mean Fraction < 0.7 μm | | |
| "T" Design | "Y" Design | HOPE |
| 0.218 | 0.239 | 0.052 |
| Mean Fraction at Midpoint = 1.52 μm | | |
| "T" Design | "Y" Design | HOPE |
| 0.519 | 0.422 | 0.227 |

The comparison of mean fraction of particles <u>greater than or equal to</u> 0.7 and less than 5.8 μm shows the present device produced a significantly greater fraction of particles, approximately 20% more, in the breathable range. The comparison of mean fraction of particles ≥ 5.8 μm shows that the present device/method produced significantly less particles, approximately 34% less, of larger particles not able to be delivered to the lungs. In addition, the device/method produced about 15% more particles < 0.7 μm, which could provide a benefit to some patients even though these sized particles are not typically deposited. For example, these size particles may actually be able to be delivered and deposited in lungs that possess severe inflammation (such as severe acute asthma patients) to which drugs would not normally be delivered. Finally, the device produced significantly more particles, approximately 22% more, with a midpoint diameter of 1.52 μm (as depicted from particles collected on the 1.1 μm cut off plate), and therefore should result in significantly greater alveolar drug deposition.

It is also believed that the present device/method would provide better particle delivery than the HOPE nebulizer by virtue of the difference in heliox concentration. The devices described herein can provide an undiluted, constant heliox concentration. In contrast, the HOPE nebulizer requires a currently available aerosol mask with two large openings thus producing an open system in which ambient air can be entrained. Additionally because the HOPE system requires oxygen or compressed air to operate the nebulizer, the HOPE system dilutes the helium or heliox flow at least 50%, based on using equivalent flow rates of oxygen or compressed air and helium or heliox. This results in 50% helium concentration under optimum circumstances, and even less when ambient air is entrained. Not only does this result in the delivery of highly variable helium concentrations, but this would dilute the helium below its effective concentration.

### Example 4: Heliox Nebulization System with Liquid Drug Suspension And Different Heliox Flow Rates

Additional tests were performed with the ACI described in Example 3 in order to assess the device's ability to nebulize a liquid drug suspension and to assess various heliox flow rate combinations. The "Y"-design device was used with 18 L/min heliox (80/20) flowing to each of the nebulizer and the side port to aerosolize and deliver budesonide inhalation suspension (Pulmicort Respules™). In addition, the heliox flow rate combinations shown in Table 9 were assessed with albuterol inhalation solution.

The particle sizes are expressed as 50% mass median aerodynamic diameters (MMAD).

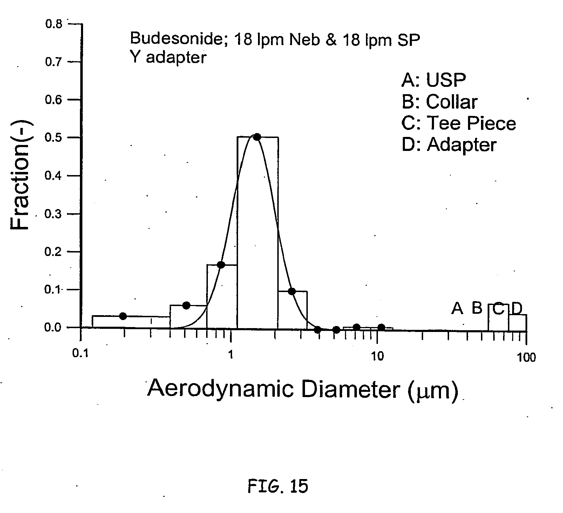

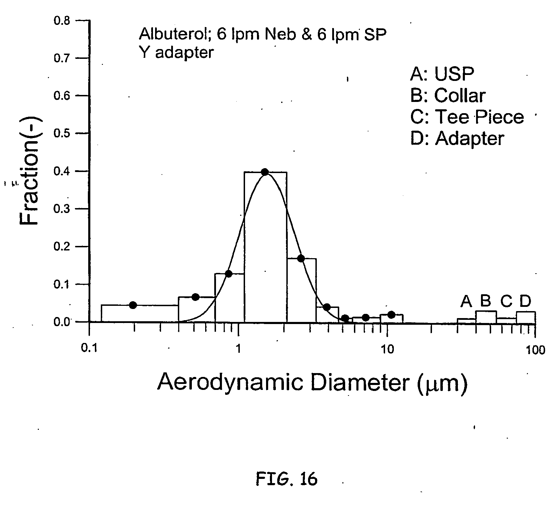

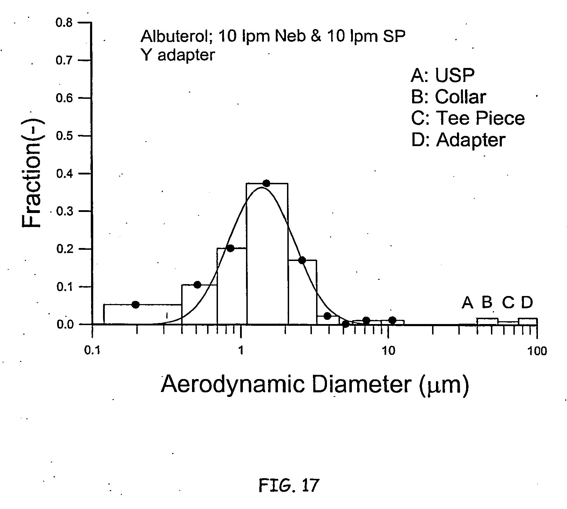

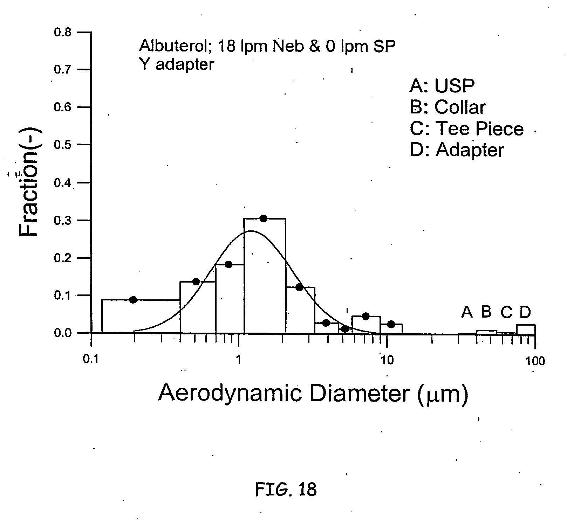

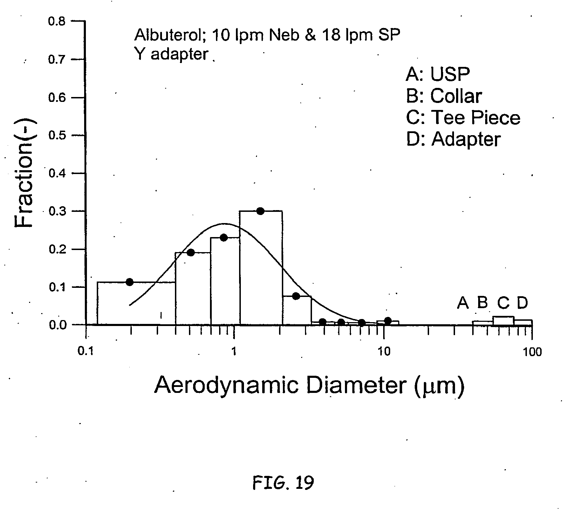

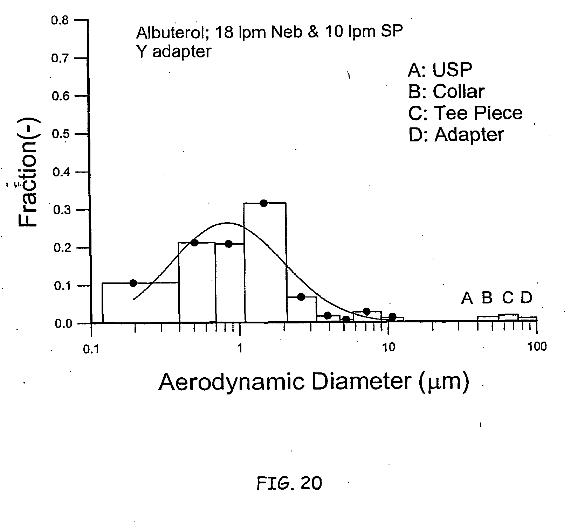

The particle size distribution for each of the tests is shown in Table 10 and FIGS. 15-20. Table 11 depicts the critical attribute characterization of the particles as previously identified for Example 3 and includes the Y configuration values from Table 8 for comparison purposes.

**Table 9. Summary of Heliox Flow Rate Combinations**

| Trial ID | Nebulizer Flowrate (L/min) | Side Port Flowrate (L/min) |
|---|---|---|
| 6-6 | 6 | 6 |
| 10-10 | 10 | 10 |
| 18-0 | 18 | 0 |
| 10-18 | 10 | 18 |
| 18-10 | 18 | 10 |

EP 1 453 562 B1

**Table 10. Particle Size**

| Stage | Cut Size (μm ) | Midpt (μm) | Budes. Fraction | 6-6 Fract | 10-10 Fract | 18-0 Fract | 10-18 Fract | 18-10 Fract |
|---|---|---|---|---|---|---|---|---|
| Adapter | | | 0.045 | 0.033 | 0.018 | 0.027 | 0.015 | 0.009 |
| Tee Piece | | | 0.071 | 0.015 | 0.009 | 0.005 | 0.024 | 0.016 |
| Collar | | | 0.000 | 0.034 | 0.018 | 0.011 | 0.011 | 0.012 |
| Throat (USP) | > 9.00 | 24.49 | 0.000 | 0.013 | 0.002 | 0.002 | 0.001 | 0.001 |
| 0 | 9.00 | 10.70 | 0.007 | 0.023 | 0.012 | 0.026 | 0.011 | 0.011 |
| 1 | 5.80 | 7.22 | 0.007 | 0.015 | 0.011 | 0.047 | 0.006 | 0.026 |
| 2 | 4.70 | 5.22 | 0.000 | 0.013 | 0.002 | 0.013 | 0.007 | 0.006 |
| 3 | 3.30 | 3.94 | 0.000 | 0.042 | 0.023 | 0.029 | 0.008 | 0.016 |
| 4 | 2.10 | 2.63 | 0.101 | 0.171 | 0.171 | 0.124 | 0.077 | 0.066 |
| 5 | 1.10 | 1.52 | 0.507 | 0.399 | 0.374 | 0.307 | 0.301 | 0.314 |
| 6 | 0.70 | 0.88 | 0.170 | 0.130 | 0.202 | 0.184 | 0.232 | 0.207 |
| 7 | 0.40 | 0.53 | 0.061 | 0.067 | 0.106 | 0.137 | 0.192 | 0.211 |
| Filter | <0.40 | <0.40 | 0.031 | 0.045 | 0.053 | 0.088 | 0.113 | 0.106 |

**Table 11: Comparison of Critical Particle Size Fractions**

| 0.7 < Mean Fraction < 5.8 μm | | | | | | |
|---|---|---|---|---|---|---|
| Budesonide 18-18 lpm | 6-6 lpm | 10-10 lpm | 18-0 lpm | 10-18 lpm | 18-10 lpm | 18-18 lpm |
| 0.778 | 0.755 | 0.773 | 0.658 | 0.626 | 0.609 | 0.657 |
| Mean Fraction > 5.8 μm | | | | | | |
| Budesonide 18-18 lpm | 6-6 lpm | 10-10 lpm | 18-0 lpm | 10-18 lpm | 18-10 lpm | 18-18 lpm |
| 0.130 | 0.134 | 0.068 | 0.118 | 0.068 | 0.075 | 0.105 |
| Mean Fraction < 0.7 μm | | | | | | |
| Budesonide 18-18 lpm | 6-6 lpm | 10-10 lpm | 18-0 lpm | 10-18 lpm | 18-10 lpm | 18-18 lpm |
| 0.092 | 0.112 | 0.159 | 0.224 | 0.306 | 0.317 | 0.239 |
| Mean Fraction at Midpoint =1.52 μm | | | | | | |
| Budesonide 18-18 lpm | 6-6 lpm | 10-10 lpm | 18-0 lpm | 10-18 lpm | 18-10 lpm | 18-18 lpm |
| 0.507 | 0.399 | 0.374 | 0.307 | 0.301 | 0.314 | 0.422 |
| All columns not labeled with "Budesonide" were tested using Albuterol. | | | | | | |

Modifications and variations of the devices are possible within the scope of the appended claims.

**Claims**

1. A medical device (10) for using heliox gas to deliver a drug to the lungs of a patient in need thereof comprising:

an aerosolization subassembly (12) which comprises:

- a gas inlet (20) for connection to a first heliox gas source,

- a drug reservoir (22) for containing a drug to be administered,

- an atomization means (21, 23) for forming an aerosol of particles or droplets dispersed in a heliox driving gas received from the first heliox gas source, wherein the particles or droplets comprise the drug, and

- a discharge outlet (24) for discharging the aerosol;

a gas mask (14) which can be secured over a patient's mouth and nose, the gas mask comprising a source gas aperture;

a secondary gas inlet (28) for connection to a second heliox gas source;

a branched conduit means (18) which comprises a first inlet port (32), a second inlet port, (34) and an outlet port (36), wherein the first inlet port is in communication with the discharge outlet of the aerosolization subassembly, the second inlet port is in communication with the secondary gas inlet, and the outlet port is in communication with the source gas aperture of the gas mask;

a source of compressed heliox gas connected to the gas inlet of the aerosolization subassembly, or to the secondary gas inlet, or to both the gas inlet of the aerosolization subassembly and to the secondary gas inlet;

a reservoir bag (16) having an opening adjacent to and in communication with the second inlet port of the branched conduit means; and

at least one exhalation port in communication with the gas mask and/or with the branched conduit means, the exhalation port comprising a one-way valve to allow exhaled gases to be expelled from the device, such that the device is operable as a closed system to prevent dilution of the aerosol with ambient air before and during inhalation of the aerosol by the patient.

2. The device of claim 1, wherein the source of compressed heliox gas is connected to both the gas inlet of the aerosolization subassembly and the secondary gas inlet.

3. The device of claim 2, wherein the source of compressed heliox gas comprises a tank coupled to a single regulator valve (50) having two discharge outlets (54a, 54b).

4. The device of claim 1, wherein the aerosolization subassembly comprises a jet nebulizer or pneumatic nebulizer.

5. The device of claim 1, wherein the aerosolization subassembly comprises an ultrasonic nebulizer or an electrostatic nebulizer.

6. The device of claim 1, wherein the first inlet port of the branched conduit means is co-axial with the outlet port of the branched conduit means.

7. The device of claim 6, wherein the branched conduit means comprises a T-shaped conduit connector, a Y-shaped connector, or a parallel Y-shaped connector.

8. The device of claim 1, further comprising a one-way valve positioned between the branched conduit means and the secondary gas inlet, the one-way valve being operable to prevent the aerosol from flowing out through the secondary gas inlet.

9. The device of claim 1, which produces an aerosol wherein greater than 55 wt% of the drug is in the form of particles having an aerodynamic diameter of greater than or equal to 0.7 micron and less than 5.8 micron.

10. The medical device of claims 1-9, wherein the compressed heliox gas consists essentially of about 80 vol.% helium and about 20 vol.% oxygen.

11. The medical device of claims 1-10, wherein the drug reservoir comprises a drug.

12. The medical device of claim 11, wherein the drug is in a dry powder form.

13. The medical device of claim 11, wherein the drug is in a liquid form.

14. The medical device of claim 11, wherein the drug is selected from bronchodilators, anti-inflammatory agents, antibiotics, antineoplastic, and combinations thereof.

15. The medical device of claim 14, wherein the bronchodilator comprises a racemic mixture of epinephrine, an anticholinergic, or a beta agonist selected from albuterol, formoterol, salmeterol, pirbuterol, metaproterenol, terbutaline, and bitolterol mesylate.

16. The medical device of claim 14, wherein the anti-inflammatory agent is selected from corticosteroids, other steroids, cromolyn, nedocromil, and leukotriene inhibitors.

17. The medical device of claim 11, wherein the drug comprises a monoclonal antibody.

18. The medical device of claim 11, wherein the drug comprises tobramycin or doxorubicin.

**Patentansprüche**

1. Medizinische Vorrichtung (10) für die Verwendung von Heliox-Gas für die Zufuhr eines Arzneimittels zu den Lungen eines Patienten der dieses benötigt, umfassend:

    eine Aerosolisierungseinheit (12) welche umfasst:

    - einen Gaseinlass (20) zur Verbindung mit einer ersten Heliox-Gasquelle,
    - einen Arzneimittelbehälter (22) um ein zu verabreichendes Arzneimittel zu enthalten,
    - ein Atomisierungsmittel (21, 23) um ein Aerosol von Partikeln oder Tröpfchen zu bilden, die in einem Heliox-Treibgas dispergiert sind, das aus der ersten Heliox-Gasquelle erhalten wird, wobei die Partikel oder Tröpfchen das Arzneimittel enthalten und
    - einen Abgabeauslass (24) um das Aerosol abzugeben;

    eine Gasmaske (14), die über Mund und Nase eines Patienten befestigt werden kann, wobei die Gasmaske eine Gasöffnung umfasst;
    einen sekundären Gaseinlass (28) zur Verbindung mit einer zweiten Heliox-Gasquelle;
    eine verzweigte Leitung (18) welche einen ersten Einlassanschluss (32), einen zweiten Einlassanschluss (34) und einen Auslassanschluss (36) umfasst, wobei der erste Einlassanschluss mit dem Abgabeauslass der Aerosolisierungseinheit in Kommunikation steht, der zweite Einlassanschluss mit dem sekundären Gaseinlass in Kommunikation steht und der Auslassanschluss mit der Gasöffnung der Gasmaske in Kommunikation steht;
    eine Quelle an komprimiertem Heliox-Gas, die mit dem Gaseinlass der Aerosolisierungseinheit oder dem sekundären Gaseinlass oder sowohl dem Gaseinlass der Aerosolisierungseinheit als auch dem sekundären Gaseinlass verbunden ist;
    eine Behältertasche (16), die eine Öffnung aufweist, die benachbart zu und in Kommunikation mit dem zweiten Einlassanschluss der verzweigten Leitung ist; und
    wenigstens einen Ausatmungsanschluss in Kommunikation mit der Gasmaske und/oder mit der verzweigten Leitung, wobei der Ausatmungsanschluss ein Einwegventil umfasst, um zu ermöglichen, das ausgeatmete Gase aus der Vorrichtung ausgestoßen werden, sodass die Vorrichtung als geschlossenes System arbeiten kann, um eine Verdünnung des Aerosols mit Umgebungsluft vor und nach Inhalation des Aerosols durch den Patienten zu vermeiden.

2. Vorrichtung nach Anspruch 1, wobei die Quelle an komprimiertem Heliox-Gas sowohl mit dem Gaseinlass der Aerosolisierungseinheit als auch mit dem sekundären Gaseinlass verbunden ist.

3. Vorrichtung nach Anspruch 2, wobei die Quelle an komprimiertem Helioxgas einen Tank umfasst, der mit einem einzelnen Regelventil (50) verbunden ist, das zwei Abgabeauslässe (54a, 54b) aufweist.

4. Vorrichtung nach Anspruch 1, wobei die Aerosolisierungseinheit einen Düsenzerstäuber oder pneumatischen Zerstäuber umfasst.

5. Vorrichtung Anspruch 1, wobei die Aerosolisierungseinheit einen Ultraschall-Zerstäuber oder einen elektrostatischen Zerstäuber umfasst.

6. Vorrichtung nach Anspruch 1, wobei der erste Einlassanschluss der verzweigten Leitung koaxial zu dem Auslassanschluss der verzweigten Leitung ist.

**7.** Vorrichtung nach Anspruch 6, wobei die verzweigte Leitung einen T-förmigen Leitungsverbinder, einen Y-förmigen Verbinder oder einen parallel Y-förmigen Verbinder umfasst.

**8.** Vorrichtung nach Anspruch 1, weiter umfassend ein Einwegventil, das zwischen der verzweigten Leitung und dem sekundären Gaseinlass positioniert ist, wobei das Einwegventil funktionsfähig ist um zu verhindern, dass Aerosol durch den sekundären Gaseinlass ausströmt.

**9.** Vorrichtung nach Anspruch 1, welche ein Aerosol produziert, in dem mehr als 55 Gew.-% des Arzneimittels in Form von Partikeln mit einem aerodynamischen Durchmesser von mehr als oder gleich 0,7 $\mu$m und weniger als 5,8 $\mu$m vorliegt.

**10.** Medizinische Vorrichtung nach einem der Ansprüche 1-9, wobei das komprimierte Heliox-Gas im Wesentlichen aus etwa 80 Vol.-% Helium und etwa 20 Vol.-% Sauerstoff besteht.

**11.** Medizinische Vorrichtung nach einem der Ansprüche 1-10, wobei der Arzneimittelbehälter ein Arzneimittel umfasst.

**12.** Medizinische Vorrichtung nach Anspruch 11, wobei das Arzneimittel in trockener Pulverform vorliegt.

**13.** Medizinische Vorrichtung nach Anspruch 11, wobei das Arzneimittel in flüssiger Form vorliegt.

**14.** Medizinische Vorrichtung nach Anspruch 11, wobei das Arzneimittel ausgewählt ist aus Bronchodilatoren, antientzündlichen Mitteln, Antibiotika, anti-neoplastischen Mitteln und Kombinationen davon.

**15.** Medizinische Vorrichtung nach Anspruch 14, wobei der Bronchodilator ein razemisches Gemisch aus Epinephrin, einem Aniticholinergikum, oder einem beta-Agonisten umfasst, ausgewählt aus Albuterol, Formoterol, Salmeterol, Pirbuterol, Metaproterenol, Terbutaline, und Bitolterol-Mesylat.

**16.** Medizinische Vorrichtung nach Anspruch 14, wobei das anti-entzündliche Mittel ausgewählt ist aus Cortikosteroiden, anderen Steroiden, Cromolyn, Nedocromil und Leukotrien-Inhibitoren.

**17.** Medizinische Vorrichtung nach Anspruch 11, wobei der Wirkstoff einen monoklonalen Antikörper umfasst.

**18.** Medizinische Vorrichtung nach Anspruch 11, wobei das Arzneimittel Tobramycin oder Doxorubicin umfasst.

## Revendications

**1.** Dispositif médical (10) pour utiliser du gaz héliox afin de délivrer un médicament dans les poumons d'un patient qui en a besoin, comprenant :

un sous-ensemble d'aérosol (12) qui comprend :

une entrée de gaz (20) pour le raccordement à une première source de gaz héliox,
un réservoir de médicament (22) pour contenir un médicament à administrer,
des moyens de pulvérisation (21, 23) pour former un aérosol de particules ou de gouttelettes dispersées dans un gaz d'entraînement d'héliox reçu de la première source de gaz héliox, dans lequel les particules ou gouttelettes comprennent le médicament, et
une sortie de décharge (24) pour décharger l'aérosol ;
un masque à gaz (14) qui peut être fixé sur la bouche et le nez d'un patient, le masque à gaz comprenant une ouverture de gaz source ;
une entrée de gaz secondaire (28) pour le raccordement à une deuxième source de gaz héliox ;
des moyens de conduit ramifiés (18) qui comprennent un premier orifice d'entrée (32), un deuxième orifice d'entrée (34) et un orifice de sortie (36), dans lequel le premier orifice d'entrée est en communication avec la sortie de décharge du sous-ensemble d'aérosol, le deuxième orifice d'entrée est en communication avec l'entrée de gaz secondaire et l'orifice de sortie est en communication avec l'ouverture de gaz de source du masque à gaz ;
une source de gaz héliox comprimé raccordée à l'entrée de gaz du sous-ensemble d'aérosol ou à l'entrée de gaz secondaire ou à la fois à l'entrée de gaz du sous-ensemble d'aérosol et à l'entrée de gaz secondaire ;

un sachet formant réservoir (16) ayant une ouverture adjacente à et en communication avec le deuxième orifice d'entrée des moyens de conduit ramifiés ; et

au moins un orifice d'expiration en communication avec le masque à gaz et/ou avec les moyens de conduit ramifiés, l'orifice d'expiration comprenant une valve à une voie pour permettre aux gaz expirés d'être expulsés du dispositif, de sorte que le dispositif peut fonctionner comme un système fermé pour empêcher la dilution de l'aérosol avec l'air ambiant avant et pendant l'inhalation de l'aérosol par le patient.

2. Dispositif selon la revendication 1, dans lequel la source de gaz héliox comprimé est raccordée à la fois à l'entrée de gaz du sous-ensemble d'aérosol et à l'entrée de gaz secondaire.

3. Dispositif selon la revendication 2, dans lequel la source de gaz héliox comprimé comprend un réservoir couplé à une valve de régulation unique (50) ayant deux sorties de décharge (54a, 54b).

4. Dispositif selon la revendication 1, dans lequel le sous-ensemble d'aérosol comprend un vaporisateur à jet ou un vaporisateur pneumatique.

5. Dispositif selon la revendication 1, dans lequel le sous-ensemble d'aérosol comprend un vaporisateur à ultrasons ou un vaporisateur électrostatique.

6. Dispositif selon la revendication 1, dans lequel le premier orifice d'entrée des moyens de conduit ramifiés est coaxial avec l'orifice de sortie des moyens de conduit ramifiés.

7. Dispositif selon la revendication 6, dans lequel les moyens de conduit ramifiés comprennent un connecteur de conduit en forme de T, un connecteur en forme de Y ou un connecteur en forme de Y parallèle.

8. Dispositif selon la revendication 1, comprenant en outre une valve à une voie positionnée entre les moyens de conduit ramifiés et l'entrée de gaz secondaire, la valve à une voie pouvant être actionnée pour empêcher l'aérosol de sortir par l'entrée de gaz secondaire.

9. Dispositif selon la revendication 1, qui produit un aérosol dans lequel plus de 55% en poids du médicament se présentent sous la forme de particules ayant un diamètre aérodynamique supérieur ou égal à 0,7 micron et inférieur à 5,8 microns.

10. Dispositif médical selon les revendications 1 à 9, dans lequel le gaz héliox comprimé se compose essentiellement d'environ 80% en volume d'hélium et d'environ 20% en volume d'oxygène.

11. Dispositif médical selon les revendications 1 à 10, dans lequel le réservoir de médicament comprend un médicament.

12. Dispositif médical selon la revendication 11, dans lequel le médicament se présente sous forme de poudre sèche.

13. Dispositif médical selon la revendication 11, dans lequel le médicament se présente sous une forme liquide.

14. Dispositif médical selon la revendication 11, dans lequel le médicament est choisi parmi les bronchodilatateurs, les anti-inflammatoires, les antibiotiques, les anticancéreux et leurs combinaisons.

15. Dispositif médical selon la revendication 14, dans lequel le bronchodilatateur comprend un mélange racémique d'adrénaline, un anticholinergique ou un bêta-agoniste choisi parmi l'albutérol, le formotérol, le salmétérol, le pirbutérol, le métaprotérénol, la terbutaline et le mésylate de bitoltérol.

16. Dispositif médical selon la revendication 14, dans lequel l'anti-inflammatoire est choisi parmi les corticostéroïdes, les autres stéroïdes, l'acide cromoglycique, le nédocromil et les inhibiteurs de leucotriène.

17. Dispositif médical selon la revendication 11, dans lequel le médicament comprend un anticorps monoclonal.

18. Dispositif médical selon la revendication 11, dans lequel le médicament comprend de la tobramycine ou de la doxorubicine.

FIG. 1

FIG. 2

FIG. 3

FIG. 4

FIG. 5

FIG. 6

FIG. 7

FIG. 8

FIG. 9

10 lpm Air

Tee    Collar    Throat

18 lpm Heliox

Tube

HOPE

Impactor

Top-Trak

28.3 L/min

Vacuum

FIG. 10

FIG. 11

**Mean RQN "Y" Particle Size Data**

A: USP
B: Collar
C: Tee Piece
D: Adapter

FIG. 12

FIG.13

EP 1 453 562 B1

50

50

53

54a

54b

52

FIG. 14A

FIG. 14B

34

FIG. 15

FIG. 16

Albuterol; 10 lpm Neb & 10 lpm SP
Y adapter

A: USP
B: Collar
C: Tee Piece
D: Adapter

FIG. 17

FIG. 18

FIG. 19

FIG. 20

GAS MASK

EXHALATION PORT / ONE WAY VALVE

ONE-WAY VALVE

RESERVOIR BAG

AEROSOLIZATION SUBASSEMBLY

FIG. 21

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Barach.** *Proc. Soc. Exp. Biol. Med,* 1934, vol. 32, 462-64
- **Manthous et al.** Heliox improves pulsus paradoxus and peak expiratory flow in nonintubated patients with severe asthma. *Am. J. Respir. Crit. Care Med.,* 1995, vol. 151, 310-14
- **Kass ; Castriotta.** Heliox therapy in acute severe asthma. *Chest,* 1995, vol. 107, 757-60
- **Gluck et al.** Helium-oxygen mixtures in intubated patients with status asthmaticus and respiratory acidosis. *Chest,* 1990, vol. 98, 693-98
- **Ball et al.** *Clin. Intensive Care,* 2001, vol. 12 (3), 105-13
- **Manthous et al.** *Respiratory Care,* 1997, vol. 42 (11), 1034-42
- **Nie et al.** *Am. J. Physiol. Heart Circ. Physiol,* 2001, vol. 280, H1875-1881
- **Habib et al.** Effect of helium-oxygen on delivery of albuterol in a pediatric volume cycled lung model. *Pharmacotherapy,* 1999, vol. 19, 143-49
- **Goode et al.** Improvement in aerosol delivery with helium-oxygen mixtures during mechanical ventilation. *Am J Respir Crit Care Med,* 2001, vol. 163, 109-14
- **Svartengren et al.** *Experimental Lung Research,* 1989, vol. 15, 575-85
- **Anderson et al.** *Am. Rev. Respir. Dis.,* 1993, vol. 147, 524-28
- **Henderson et al.** *Ann. Emerg. Med.,* 1999, vol. 33, 141-46
- **deBoisblanc et al.** *Crit. Care Med.,* 2000, vol. 28 (9), 3177-80
- **FW MOLER ; CE JOHNSON ; C VAN LAANEN ; JM PALMISANO.** CONTINUOUS VERSUS INTERMITTENT NEBULIZED TERBUTALINE: PLASMA LEVELS AND EFFECTS. *AM J. RESPIR. CRIT. CARE MED,* March 1995, vol. 151 (3), 602-606
- American Thoracic Society: Standardization of spirometry-1994 update. *Am. J. Respir. Crit. Care Med,* 1995, vol. 152, 1107-36
- American Thoracic Society: Standardization of spirometry-1994 update. *Am. J Respir. Crit. Care Med.,* 1995, vol. 152, 1107-36